# EUROPEAN PATENT APPLICATION

(11) **EP 3 382 396 A1**
(43) Date of publication of application: **03.10.2018**
(21) Application number: 16868722.6
(22) Date of filing: 25.11.2016
(51) Int. Cl.: G01N 35/02, G01N 35/00, G01N 35/10

(54) **SPECIMEN ANALYSIS DEVICE**

(30) Priority: 27.11.2015 JP 2015232374
(71) Applicant: Sysmex Corporation, Kobe-shi, Hyogo 651-0073 (JP)
(72) Inventor: KATSUMI, Hironori, Kobe-shi Hyogo 651-0073 (JP); SAITO, Shunsuke, Kobe-shi Hyogo 651-0073 (JP); ODA, Kohei, Kobe-shi Hyogo 651-0073 (JP)
(74) Representative: Hoffmann Eitle
(86) International application number: PCT/JP2016/085993
(87) International publication number: WO 2017/090779

(57) **Abstract**

Provided is a sample analyzer that can suppress an increase in the installation area of the sample analyzer while maintaining high sample processing capability and also simplify preparation work. The sample analyzer (10) includes: a first reagent dispensing unit (12) that aspirates a reagent from a first aspirating position (23a); a second reagent dispensing unit (13) that aspirates a reagent from a second aspirating position (23b); a first detection unit (14) that measures a measurement specimen containing the reagent dispensed by the first reagent dispensing unit (12) and a sample; a second detection unit (15) that measures a measurement specimen containing the reagent dispensed by the second reagent dispensing unit (13) and a sample; and a controller (26) that controls a reagent table (23) so that the reagent related to a measurement item set so as to use the first reagent dispensing unit (12) is transferred to the first aspirating position (23a), and the reagent related to a measurement item set so as to use the second reagent dispensing unit (13) is transferred to the second aspirating position (23b).

## Description

### TECHNICAL FIELD

The invention relates to a sample analyzer which analyzes a specimen prepared by mixing a sample with a reagent.

### BACKGROUND ART

A sample analyzer is known which analyzes a specimen prepared by mixing a sample with a reagent. Patent Document 1 describes an analysis system which analyzes samples by using a coagulation time method and which includes an analyzer and an extension analyzer with substantially the same configurations to improve sample processing capability.

### PRIOR ART DOCUMENT

### PATENT DOCUMENT

Patent Document 1: Japanese Patent Application Publication No. 2007-10562

### SUMMARY

### PROBLEMS TO BE SOLVED BY THE INVENTION

There is a demand for a sample analyzer with a smaller size and an improved sample processing capability. In the configuration of the aforementioned Patent Document 1, although the sample processing capability is improved, the installation area of the entire apparatus is large because the analyzer and the extension analyzer with substantially the same configurations are arranged. Moreover, in order to obtain accurate measurement results in a blood coagulation test among various clinical tests, the measurement needs to be performed in accordance with predetermined exact conditions regarding the amount of reagent dispensed and the temperature of the reagent dispensed. Accordingly, when two analyzers in an analysis system which analyzes samples by using the coagulation time method measure the samples for the same measurement item, measurement accuracy may differ between the two analyzers. In order to reduce this difference, the user must perform preparation work such as preparation of calibration curve and quality control in substantially the same way for the two analyzers.

### MEANS FOR SOLVING THE PROBLEMS

A main aspect of the present invention relates to a sample analyzer. A sample analyzer according to the aspect comprises: a reagent table on which reagent containers each holding a reagent are placed and that transfers the placed reagent containers to a first aspirating position and a second aspirating position; a first reagent dispensing unit that aspirates the reagent from the reagent container transferred to the first aspirating position, heats the aspirated reagent, and dispenses the heated reagent into reaction containers; a second reagent dispensing unit that aspirates the reagent from the reagent container transferred to the second aspirating position, heats the aspirated reagent, and dispenses the heated reagent into the reaction containers; a detection unit including holders that hold the reaction containers each holding a measurement specimen containing a sample and the reagent dispensed by the first reagent dispensing unit and the reaction containers each holding a measurement specimen containing a sample and the reagent dispensed by the second reagent dispensing unit, and detectors that are provided for the respective holders; and a controller that controls the reagent table such that the reagent table transfers the reagent container, holding the reagent relating to a measurement item for which the first reagent dispensing unit is set to be used, to the first aspirating position and transfers the reagent container, holding the reagent relating to a measurement item for which the second reagent dispensing unit is set to be used, to the second aspirating position.

### EFFECT OF THE INVENTION

The present invention may suppress an increase in the installation area of the sample analyzer while maintaining high sample processing capability and also simplify preparation work.

### BRIEF DESCRIPTION OF DRAWINGS

FIG. 1 is a view schematically illustrating an overall configuration of a sample analyzer according to one or more embodiments;
FIG. 2 is a view illustrating a specific configuration of a sample analyzer according to one or more embodiments;
FIG. 3A is a side view illustrating a cross section of a reaction container according to one or more embodiments, and FIGS. 3B and 3C are views schematically illustrating configurations of transfer units according to one or more embodiments;
FIG. 4A is a view schematically illustrating a configuration of a transfer unit according to one or more embodiments, and FIGS. 4B and 4C are views schematically illustrating a configuration around an arm of a transfer unit according to one or more embodiments;
FIG. 5A is a view illustrating configurations of a reaction container housing unit and a reaction container supplying unit according to one or more embodiments, and FIG. 5B is a view illustrating a configuration of a reaction container supplying unit according to one or more embodiments;
FIG. 6 is a view schematically illustrating configurations of reagent dispensing units, a reagent table, a support unit, and a base according to one or more embodiments;
FIG. 7 is a view schematically illustrating a configuration around a heating table according to one or more embodiments;
FIG. 8A is a view schematically illustrating a configuration of a light emitting unit according to one or more embodiments, and FIG. 8B is a view illustrating a filter portion from a light incidence side;
FIG. 9A is a cross-sectional view obtained by cutting a detector according to one or more embodiments along a plane parallel to a Y-Z plane, and FIG. 9B is a cross-sectional view obtained by cutting a detector according to one or more embodiments along a plane parallel to an X-Z plane;
FIG. 10A is a perspective view schematically illustrating configurations of housings according to one or more embodiments, and FIG. 10B is a schematic view illustrating the configurations of housings according to one or more embodiments are viewed from above;
FIG. 11 is a block diagram illustrating a circuit configuration of a measurement unit according to one or more embodiments;
FIG. 12A is a block diagram illustrating a configuration of an information processing apparatus according to one or more embodiments, and FIG. 12B is a conceptual diagram illustrating association information according to one or more embodiments;
FIGS. 13A and 13B are views illustrating transfer paths of a sample and a reaction container in a first aspirating mode according to one or more embodiments;
FIGS. 14A and 14B are views illustrating transfer paths of a sample and a reaction container in a second aspirating mode according to one or more embodiments;
FIGS. 15A and 15B are views illustrating transfer paths of a sample and a reaction container according to one or more embodiments;
FIG. 16 is a flowchart illustrating processing performed by an information processing apparatus according to one or more embodiments;
FIG. 17 is a flowchart illustrating processing performed by a measurement unit according to one or more embodiments;
FIG. 18 is a flowchart illustrating measurement processing performed by a measurement unit according to one or more embodiments;
FIG. 19 is a flowchart illustrating measurement processing performed by a measurement unit according to one or more embodiments;
FIG. 20 is a flowchart illustrating measurement processing performed by a measurement unit according to one or more embodiments;
FIG. 21A is a flowchart illustrating processing performed by an information processing apparatus according to one or more embodiments to receive setting of association information in which measurement items and reagent dispensing units used to dispense reagents are associated with one another, FIG. 21B is a view illustrating a configuration of a receiving portion which receives setting of a reagent dispensing unit to be used for each measurement item according to one or more embodiments.

### DETAILED DESCRIPTION

A sample analyzer of one or more embodiments may be a blood coagulation analyzer which analyzes coagulability of blood by emitting light to a measurement specimen, prepared by adding a reagent to a sample, and analyzing obtained transmitted or scattered light by using a coagulation method, a chromogenic substrate method, immunonephelometry, or an agglutination method. The sample analyzed in one or more embodiments may be plasma or serum separated from the blood.

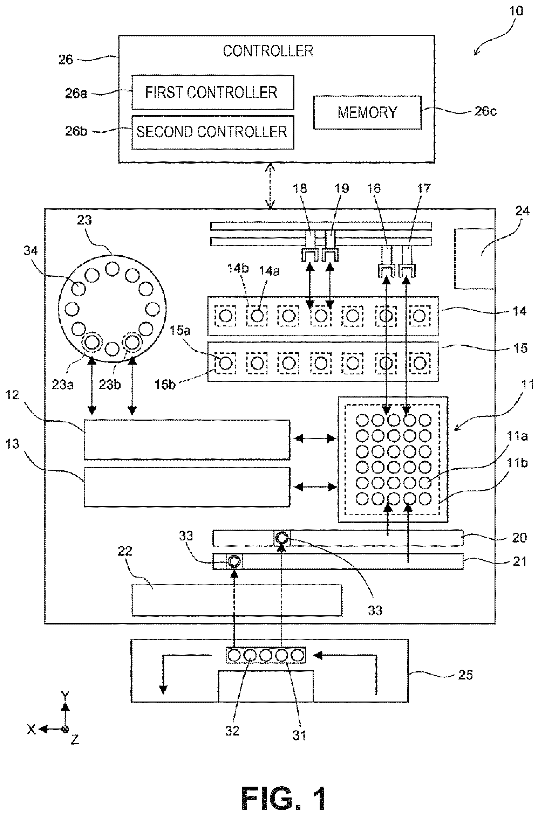

An outline of the sample analyzer of one or more embodiments is described with reference to FIG. 1. In FIG. 1, the X, Y, and Z axes are orthogonal to one another. An X-axis positive direction corresponds to the leftward direction, a Y-axis positive direction corresponds to the rearward direction, and a Z-axis positive direction corresponds to the vertically-downward direction. Note that the X, Y, and Z axes are set as in FIG. 1 in the other drawings.

The sample analyzer 10 includes a heating table 11, a first reagent dispensing unit 12, a second reagent dispensing unit 13, a first detection unit 14, a second detection unit 15, a first transfer unit 16, a second transfer unit 17, a first discarding transfer unit 18, a second discarding transfer unit 19, a first sample transfer unit 20, a second sample transfer unit 21, a sample dispensing unit 22, a reagent table 23, a discarding unit 24, a transporter 25, and a controller 26. The first transfer unit 16, the second transfer unit 17, the first discarding transfer unit 18, and the second discarding transfer unit 19 each include a catcher for gripping a reaction container 33 and a mechanism for driving the catcher.

The transporter 25 transfers a sample rack 31 holding sample containers 32. The sample dispensing unit 22 aspirates the samples contained in the sample containers 32 and discharges the aspirated samples into the reaction containers 33. The first sample transfer unit 20 and the second sample transfer unit 21 transfer the reaction containers 33 in which the samples are dispensed to the heating table 11.

The heating table 11 includes holding holes 11 a and a heater 11b. The holding holes 11a hold the reaction containers 33 transferred by the first sample transfer unit 20 or the second sample transfer unit 21. The heater 11b heats the reaction containers 33 held in the holding holes 11a to 37°C. The time of heating the reaction containers 33 with the heating table 11 is determined for each measurement item to obtain accurate measurement results in the first detection unit 14 and the second detection unit 15. For example, when Thrombocheck (registered trademark) PT plus manufactured by Sysmex Corporation is used for a measurement item PT, the heating time is three minutes. For example, when Thrombocheck (registered trademark) APTT manufactured by Sysmex Corporation is used for the measurement item APTT, the heating time is three minutes. For example, when LIAS AUTO (registered trademark) D dimer neo manufactured by Sysmex Corporation is used for the measurement item D dimer, the heating time is three minutes.

The first reagent dispensing unit 12 aspirates the reagent from one of reagent containers 34 held in the reagent table 23, heats the aspirated reagent, and discharges the heated reagent into the reaction container 33 at a predetermined position on a path in which the first transfer unit 16 transfers the reaction container 33 from the heating table 11 to a first holder 14a of the first detection unit 14. The second reagent dispensing unit 13 aspirates the reagent from one of the reagent containers 34 held in the reagent table 23, heats the aspirated reagent, and discharges the heated reagent into the reaction container 33 at a predetermined position on a path in which the second transfer unit 17 transfers the reaction container 33 from the heating table 11 to a second holder 15a of the second detection unit 15. The amount and temperature of reagent discharged into each reaction container 33 need to be exact to obtain correct measurement results.

When reagents needs to be dispensed also into the reaction containers 33 heated by the heating table 11 based on the measurement item, the first reagent dispensing unit 12 and the second reagent dispensing unit 13 aspirate the corresponding reagents from the reagent containers 34 held in the reagent table 23, heats the aspirated reagents, and dispense the heated reagents into the reaction containers 33 heated in the heating table 11.

The first detection unit 14 includes the first holders 14a and first detectors 14b provided to correspond to the respective first holders 14a. The second detection unit 15 includes the second holders 15a and second detectors 15b provided to correspond to the respective second holders 15a.

The first holders 14a and the second holders 15a have similar configurations and the first detectors 14b and the second detectors 15b have similar configurations. The first detectors 14b detect signals for analysis obtained from the measurement specimens in the reaction containers 33 held by the first holders 14a. The second detectors 15b detect signals for analysis obtained from the measurement specimens in the reaction containers 33 held by the second holders 15a.

The first detectors 14b and the second detectors 15b emit light on side surfaces of the reaction containers 33 held by the first holders 14a and the second holders 15a, receive the transmitted or scattered light with photodetectors, and output detection signals depending on the amounts of received light. The first detectors 14b and the second detectors 15b output the detection signals to a second controller 26b of the controller 26. The first detectors 14b and the second detectors 15b each emit light having various wavelengths to the corresponding reaction container 33 in a time-divided manner and output the detection signals corresponding to the light having the respective wavelengths to the second controller 26b. The first detectors 14b and the second detectors 15b are configured to be capable of performing measurement corresponding to all measurement items to be performed in the sample analyzer 10. Note that, since a change in reaction occurring in the measurement specimen in each reaction container 33 varies depending on the samples, the time necessary for the completion of the measurement varies depending on the samples.

The first transfer unit 16 transfers each of the reaction containers 33 held in the holding holes 11a of the heating table 11 to one of the first holders 14a. The second transfer unit 17 transfers each of the reaction containers 33 held in the holding holes 11a of the heating table 11 to one of the second holders 15a.

The first discarding transfer unit 18 and the second discarding transfer unit 19 dispose of the reaction containers 33 for which the measurement is completed in the first detection unit 14 and the second detection unit 15 as needed. The first discarding transfer unit 18 transfers, to the discarding unit 24, the reaction containers 33 which are held by the first holders 14a and which are to be discarded. The second discarding transfer unit 19 transfers, to the discarding unit 24, the reaction containers 33 which are held by the second holder 15a and which are to be discarded. The first discarding transfer unit 18 and the second discarding transfer unit 19 can transfer, to the discarding unit 24, the reaction containers 33 to be discarded in the first detection unit 14 and the second detection unit 15 in parallel.

The first sample transfer unit 20 and the second sample transfer unit 21 transfer, to the heating table 11, the reaction containers 33 into which the sample dispensing unit 22 has dispensed the samples as described above. The sample dispensing unit 22 aspirates the sample from the sample container 32 at a predetermined position in a transport path of the transporter 25 and dispenses the aspirated sample into the reaction container 33. In the reagent table 23, the reagent containers 34 holding the reagents are placed and the reagent table 23 transfers the placed reagent containers 34 to a first aspirating position 23a and a second aspirating position 23b. The discarding unit 24 includes a disposal opening. The reaction containers 33 for which the measurement has been performed and which are transferred by the first discarding transfer unit 18 and the second discarding transfer unit 19 are dropped into the disposal opening of the discarding unit 24.

The controller 26 includes a first controller 26a, the second controller 26b, and a memory 26c. The first controller 26a controls mechanisms for measurement of the measurement specimens in the sample analyzer 10. The second controller 26b analyzes the samples in the reaction containers 33 held by the first holders 14a based on the signals detected by the first detectors 14b and analyzes the samples in the reaction containers 33 held by the second holders 15a based on the signals detected by the second detectors 15b.

As described above, the first detectors 14b and the second detectors 15b output, to the second controller 26b, the detection signals obtained from the measurement specimens when emitting light having various wavelengths to the measurement specimens. The second controller 26b analyzes the samples based on the detection signals obtained for the respective wavelengths, by using the coagulation method, the chromogenic substrate method, the immunonephelometry, or the agglutination method.

For example, in the coagulation time method, each of the first detectors 14b and the second detectors 15b emits light with a wavelength of 660 nm to the corresponding measurement specimen and detects the transmitted or scattered light from the measurement specimen with a photodetector to analyze the time it takes for the fibrinogen to convert to fibrin. Measurement items of the coagulation time method include PT (prothrombin time), APTT (activated partial thromboplastin time), Fbg (fibrinogen amount), and the like. Moreover, in the chromogenic substrate method, each of the first detectors 14b and the second detectors 15b emits light with a wavelength of 405 nm to the corresponding measurement specimen and detects the transmitted or scattered light from the measurement specimen with the photodetector. Measurement items of the chromogenic substrate method include ATIII, α2-PI (α2-plasmin inhibitor), PLG (plasminogen), and the like. Furthermore, in the immunonephelometry, each of the first detectors 14b and the second detectors 15b emits light with a wavelength of 800 nm to the corresponding measurement specimen and detects the transmitted and scattered light from the measurement specimen with the photodetector. Measurement items of the immunonephelometry include D dimer, FDP, and the like. Moreover, in the platelet aggregation method, each of the first detectors 14b and the second detectors 15b emits light with a wavelength of 575 nm to the corresponding measurement specimen and detects transmitted or scattered light from the measurement specimen with the photodetector.

For example, the second controller 26b calculates the absorbance of each of the measurement specimens based on the detection signal outputted from the corresponding one of the first detectors 14b and the second detectors 15b and calculates the time it takes for the calculated absorbance to exceed a predetermined threshold, as coagulation time of the measurement specimen. The second controller 26b may obtain turbidity instead of the absorbance from the detection signal and calculate the time it takes for the turbidity to exceed a predetermined threshold, as the coagulation time of the sample. Moreover, the second controller 26b may calculate the time it takes for the detection signal outputted from each of the first detectors 14b and the second detectors 15b to exceed a predetermined threshold, as the coagulation time of the corresponding measurement specimen.

The first controller 26a controls the reagent table 23 such that the reagent table 23 transfers the reagent container 34, holding the reagent relating to the measurement item for which the first reagent dispensing unit 12 is set to be used, to the first aspirating position 23a and transfers the reagent container 34, holding the reagent relating to the measurement item for which the second reagent dispensing unit 13 is set to be used, to the second aspirating position 23b. Association information in which the measurement items and the reagent dispensing units to be used are associated with one another is stored in advance in the memory 26c of the controller 26. The first controller 26a determines the reagent dispensing unit to be used based on the association information stored in the memory 26c when performing the measurement according to the measurement item.

The first reagent dispensing unit 12 aspirates the reagent, corresponding to the measurement item of the sample contained in the reaction container 33, from the reagent container 34 transferred to the first aspirating position 23a and dispenses the aspirated reagent into the reaction container 33. The second reagent dispensing unit 13 aspirates the reagent, corresponding to the measurement item of the sample contained in the reaction container 33, from the reagent container 34 transferred to the second aspirating position 23b and dispenses the aspirated reagent into the reaction container 33.

For example, when the measurement is performed for the measurement item APTT based on the aforementioned coagulation time method, the first reagent dispensing unit 12 and the second reagent dispensing unit 13 aspirate a first reagent containing phospholipid and activator from the reagent container 34 holding the first reagent and dispense the aspirated first reagent into the reaction containers 33 heated by the heating table 11. The first reagent is thereby mixed with the samples. Thereafter, when the reaction containers 33 are transferred from the heating table 11 to the first detection unit 14 or the second detection unit 15, the first reagent dispensing unit 12 and the second reagent dispensing unit 13 aspirate a second reagent containing calcium salt from another reagent container 34 holding the second reagent and dispense the aspirated second reagent into the transferred reaction containers 33. The second reagent is thereby mixed with the samples.

Moreover, when the measurement is performed for the measurement item PT based on the aforementioned coagulation time method, the first reagent dispensing unit 12 and the second reagent dispensing unit 13 aspirate a reagent containing a tissue factor and calcium salt from the reagent container 34 holding the reagent and dispense the aspirated reagent into the reaction containers 33 transferred from the heating table 11 to the first detection unit 14 or the second detection unit 15. The reagent containing the tissue factor and calcium salt is thereby mixed with the samples.

The first detection unit 14 and the second detection unit 15 thus obtain the detection signals from the measurement specimens mixed with the reagent(s) corresponding to the measurement item APTT or the measurement item PT as described above and output the detection signals to the second controller 26b. As described above, the second controller 26b analyzes the time it takes for fibrinogen to convert to fibrin in each of the samples based on the detection signal received from the first detection unit 14 or the second detection unit 15 and obtains the analysis result.

Since it is necessary to wait for the measurement specimen to coagulate in the analysis based on the coagulation time method, the measurement takes time. Generally, in the analysis based on the coagulation time method, the first detectors 14b or the second detectors 15b need to continue performing the detection with the reaction containers 33 held by the first holders 14a or the second holder 15a for several minutes. Accordingly, the reaction containers 33 tend to stay in the first detection unit 14 and the second detection unit 15.

Moreover, since it is necessary to wait for the temperature of the measurement specimens to rise to a predetermined temperature in the heating table 11, the heating takes time. Generally, the reaction containers 33 need to be held in the holding holes 11a of the heating table 11 for about several minutes until the heating of the measurement specimens is completed in the heating table 11. Accordingly, the reaction containers 33 tend to stay in the heating table 11.

In the sample analyzer 10 of one or more embodiments, the first reagent dispensing unit 12 and the second reagent dispensing unit 13 dispense the reagent into the reaction containers 33 and the first detection unit 14 and the second detection unit 15 measure the measurement specimens in the reaction containers 33. This can improve the processing capability of the sample analyzer 10 compared to the case where one reagent dispensing unit and one detection unit are used.

The time of heating the reaction containers 33 with the heating table 11 is determined for each measurement item to obtain accurate measurement results in the first detection unit 14 and the second detection unit 15. In order to accurately heat the reaction containers 33 depending on the measurement item, the reaction containers 33 need to be transferred from the holding holes 11a of the heating table 11 at appropriate timing. Since the sample analyzer 10 is provided with multiple transfer units, that is the first transfer unit 16 and the second transfer unit 17 to transfer the reaction containers 33 from the heating table 11, the reaction containers 33 can be transferred from the heating table 11 at appropriate timing. The accurate heating time can be thereby achieved more easily.

Moreover, particularly in the coagulation time method, the immunonephelometry, and the platelet aggregation method, the time it takes for the measurement to complete varies depending on the samples because the change in reaction occurring in the measurement specimen is detected. Accordingly, it is difficult to accurately predict how long each reaction container 33 needs to be held by the first holder 14a or the second holder 15a. Thus, the reaction containers 33 are held by the holders long enough for the measurement to be substantially completed for any sample, and tend to stay in the first detection unit 14 and the second detection unit 15. In this regard, since the sample analyzer 10 is provided with multiple transfer units, that is the first transfer unit 16 and the second transfer unit 17 to transfer the reaction containers 33 to the first detection unit 14 and the second detection unit 15, the reaction containers 33 can be quickly transferred to the open first holders 14a and second holders 15a. This can improve the processing capability of the sample analyzer 10.

The first detectors 14b and the second detectors 15b are configured to be capable of performing measurement corresponding to all measurement items to be performed in the sample analyzer 10. The first detection unit 14 and the second detection unit 15 can thereby receive the reaction containers 33 irrespective of the measurement items. This can improve the processing capability of the sample analyzer 10.

Since the reagent table 23 is shared by the first reagent dispensing unit 12 and the second reagent dispensing unit 13, the units can be integrated compared to the case where separate reagent tables are provided respectively for the first reagent dispensing unit 12 and the second reagent dispensing unit 13. Moreover, since the heating table 11 is shared by the first transfer unit 16 and the second transfer unit 17, the units can be integrated compared to the case where separate heating tables are provided respectively for the first transfer unit 16 and the second transfer unit 17. Accordingly, an increase in the installation area of the sample analyzer 10 can be suppressed.

The first reagent dispensing unit 12 dispenses the reagent, relating to the measurement item for which the first reagent dispensing unit 12 is set to be used, into the reaction container 33 and this reaction container 33 is transferred to the first detection unit 14. The second reagent dispensing unit 13 dispenses the reagent, relating to the measurement item for which the second reagent dispensing unit 13 is set to be used, into the reaction container 33 and this reaction container 33 is transferred to the second detection unit 15. In other words, the reagent dispensing unit and the detection unit to be used are predetermined for each measurement item. Accordingly, for the same measurement item, the same reagent dispensing unit dispenses the reagent, and the amount and temperature of the reagent discharged into the reaction container 33 are thus about the same. Hence, the measurement results with substantially the same level of accuracy are obtained for the same measurement item. Preparation work for each reagent dispensing unit and each detection unit is thus unnecessary for the same measurement item. This can suppress consumption of the reagent necessary for the preparation and simplify a preparation operation.

The first transfer unit 16 and the second transfer unit 17 transfer the reaction containers 33 from the heating table 11 to the first detection unit 14 and the second detection unit 15 in parallel. Accordingly, it is possible to avoid the case where the reaction containers 33 which should be transferred from the heating table 11 to the first detection unit 14 and the second detection unit 15 wait to be transferred, and the holding holes 11a of the heating table 11 can be quickly made vacant for reception of the next reaction containers 33. This can improve the processing capability of the sample analyzer 10.

The first discarding transfer unit 18 and the second discarding transfer unit 19 can transfer the reaction containers 33 in the first detection unit 14 and the second detection unit 15 which are to be discarded to the discarding unit 24 in parallel. Thus, it is possible to avoid the case where the reaction containers 33 waiting to be discarded remain in the first detection unit 14 and the second detection unit 15, and the transfer of the reaction containers 33 from and to the first detection unit 14 and the second detection unit 15 can be smoothly performed. This can improve the processing capability of the sample analyzer 10.

The first sample transfer unit 20 and the second sample transfer unit 21 transfer the reaction containers 33 into which the samples are dispensed to the heating table 11 in parallel. Accordingly, the reaction containers 33 into which the samples from the sample containers 32 are dispensed can be efficiency transferred to the heating table 11. This can improve the processing capability of the sample analyzer 10.

The reagent dispensing units configured to dispense the reagent are not limited to two dispensing units which are the first reagent dispensing unit 12 and the second reagent dispensing unit 13 and may be three or more dispensing units. In this case, the detection units, the transfer units configured to transfer the reaction containers 33 to the detection units, and the transfer units configured to dispose of the reaction containers 33 from the detection units may be provided to correspond to the respective reagent dispensing units. The transfer units configured to transfer the reaction containers 33 into which the samples are dispensed by the sample dispensing unit 22 to the heating table 11 are not limited to two dispensing units which are the first sample transfer unit 20 and the second sample transfer unit 21 and may be three or more dispensing units.

### <Specific Configuration Example>

A specific configuration of the sample analyzer in one or more embodiments is described below.

In the following configuration example, transfer units configured to transfer the reaction containers from a heating table to detection units are also used as transfer units configured to transfer the reaction containers from the detection units to a discarding unit. In the following description, a reagent dispensed into the reaction containers when the reaction containers are transferred from the heating table to the detection units is referred to as "trigger reagent" and a reagent dispensed into the reaction containers when the reaction containers are heated in the heating table is referred to as "adjustment reagent" for the sake of convenience. The trigger reagent is a reagent for starting reaction on samples and the adjustment reagent is a reagent for promoting the reaction by the trigger reagent.

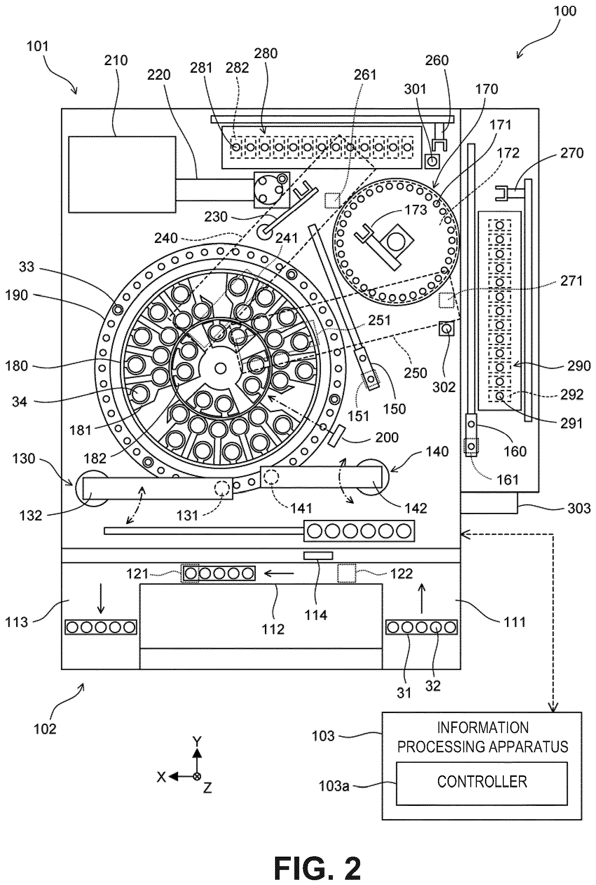

As illustrated in FIG. 2, a sample analyzer 100 includes a measurement unit 101, a transporter 102, and an information processing apparatus 103.

The transporter 102 corresponds to the transporter 25 in FIG. 1. The transporter 102 includes a rack setter 111, a rack transporter 112, a rack collector 113, and a bar code reader 114. The rack setter 111 and the rack collector 113 are connected to a right end and a left end of the rack transporter 112, respectively. The bar code reader 114 is provided behind the rack transporter 112 and is configured to be movable in a left-right direction.

A user places the sample rack 31 in which the sample containers 32 are set, on the rack setter 111. Bar codes are attached to the sample rack 31 and the sample containers 32. The transporter 102 sends the sample rack 31 placed on the rack setter 111 to the right end of the rack transporter 112 and then to the front of the bar code reader 114. The bar code reader 114 reads the bar code attached to the sample rack 31 and also reads the bar codes attached to the sample containers 32. The bar code of the sample rack 31 holds identification information for identifying the sample rack 31 and the bar codes of the sample containers 32 hold identification information for identifying the samples contained in the sample containers 32. The read identification information is sent to the information processing apparatus 103 to obtain the measurement item for the samples.

Thereafter, the transporter 102 transports the sample rack 31 and disposes the sample containers 32 one by one at a sample aspirating position 121 or a sample aspirating position 122. The samples are aspirated from the sample containers 32 at the sample aspirating positions 121, 122. When the aspiration of the samples for all sample containers 32 held in the sample rack 31 is completed, the transporter 102 transfers the sample rack 31 to the rack collector 113.

The measurement unit 101 aspirates the samples from the sample containers 32 at the sample aspirating positions 121, 122 and mixes the aspirated samples with the reagent to perform measurement.

The measurement unit 101 includes sample dispensing units 130, 140, transfer units 150, 160, a heating table 170, a reagent table 180, a reaction container table 190, a bar code reader 200, a reaction container housing unit 210, a reaction container supplying unit 220, a transfer unit 230, reagent dispensing units 240, 250, transfer units 260, 270, detection units 280, 290, disposal openings 301, 302, and a discarding unit 303.

The sample dispensing units 130, 140 each form the sample dispensing unit 22 in FIG. 1. The sample dispensing unit 130 includes a pipet 131, a turnable arm 132 having an end portion provided with the pipet 131, a not-illustrated driver for driving the arm 132, and a not-illustrated pump for aspirating and discharging the sample by using the pipet 131. Similarly, the sample dispensing unit 140 includes a pipet 141, a turnable arm 142 having an end portion provided with the pipet 141, a not-illustrated driver for driving the arm 142, and a not-illustrated pump for aspirating and discharging the sample by using the pipet 141.

The sample dispensing unit 130 aspirates the sample from the sample container 32 disposed at the sample aspirating position 121 and discharges the aspirated sample into the new reaction container 33 held in the reaction container table 190. The sample dispensing unit 140 aspirates the sample from the sample container 32 disposed at the sample aspirating position 122 or the reaction container 33 held in the reaction container table 190 and discharges the aspirated sample into the new reaction container 33 held by the transfer unit 150, 160.

The transfer units 150, 160 correspond to the first sample transfer unit 20 and the second sample transfer unit 21 in FIG. 1, respectively. The transfer units 150, 160 are configured to be movable in a front-rear direction along rails. The transfer units 150, 160 are provided with holding holes for holding the reaction containers 33.

The transfer unit 150 holds the new reaction container 33 in the holding hole and disposes the reaction container 33 at a first sample discharging position 151. As described later, the transfer unit 230 sets the new reaction container 33 on the transfer unit 150. When the sample dispensing unit 140 discharges the sample into the reaction container 33 disposed at the first sample discharging position 151, the transfer unit 150 transfers the reaction container 33 rearward and disposes the reaction container 33 near and on the left side of the heating table 170. A transfer unit 173 of the heating table 170 transfers the reaction container 33 disposed near and on the left side of the heating table 170 to one of holding holes 171 of the heating table 170.

Similarly, the transfer unit 160 holds the new reaction container 33 in the holding hole and disposes the reaction container 33 at a second sample discharging position 161. As described later, the transfer unit 260 sets the new reaction container 33 on the transfer unit 160. When the sample dispensing unit 140 discharges the sample into the reaction container 33 disposed at the second sample discharging position 161, the transfer unit 160 transfers the reaction container 33 rearward and disposes the reaction container 33 near and on the right side of the heating table 170. The transfer unit 270 transfers the reaction container 33 disposed near and on the right side of the heating table 170 to one of the holding holes 171 of the heating table 170.

Since the transfer units 150, 160 can transfer the reaction containers 33 to the heating table 170 as described above, the processing capability can be improved compared to the case where only one of the transfer units 150, 160 transfers the reaction containers 33 to the heating table 170.

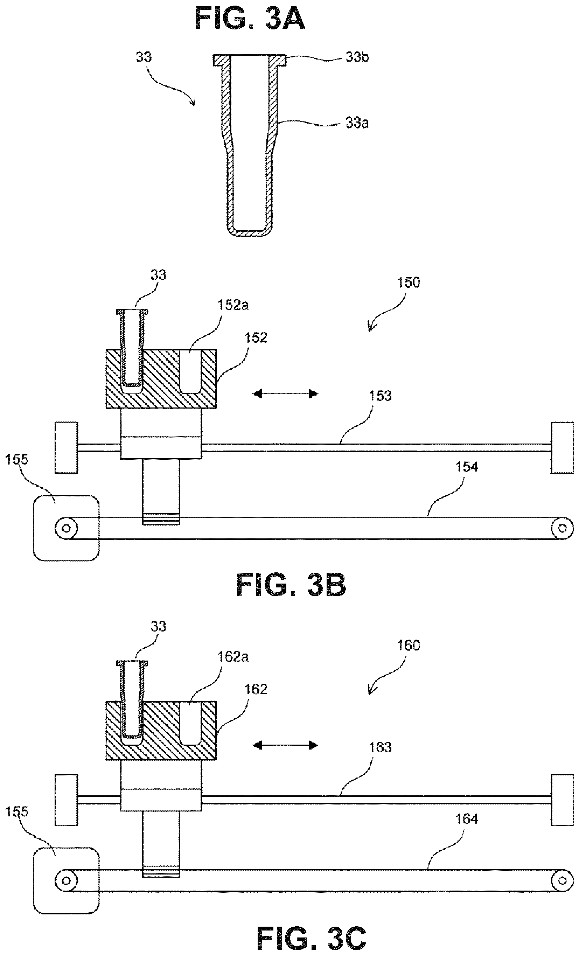

As illustrated in FIG. 3A, the reaction container 33 is a cuvette. The reaction container 33 includes a cylindrical body 33a having an opening on the upper side and a flange 33b provided in an upper portion of the body 33a. The diameter of a lower portion of the body 33a is smaller than that of the upper portion.

As illustrated in FIG. 3B, the transfer unit 150 includes a holder 152 having two holding holes 152a for holding the reaction container 33, a rail 153 configured to guide the holder 152, a belt 154 connected to the holder 152, and a motor 155 configured to drive the belt 154. In FIG. 3B, the holder 152 is illustrated in a cross-sectional view for convenience. The holding holes 152a each have such a diameter that the lower portion of the reaction container 33 can fit therein. The rail 153 is provided to extend in a transfer direction of the reaction container 33. The belt 154 is arranged parallel to the rail 153. The belt 154 is wound around a drive shaft of the motor 155 and a pulley. A lower end of the holder 152 is attached to the belt 154. The motor 155 drives the belt 154 and the holder 152 is thereby transferred while being guided by the rail 153. The reaction container 33 can be thereby transferred.

As illustrated in FIG. 3C, the transfer unit 160 also has a configuration similar to the transfer unit 150. The transfer unit 160 includes a holder 162 having two holding holes 162a for holding the reaction container 33, a rail 163 configured to guide the holder 162, a belt 164 connected to the holder 162, and a motor 165 configured to drive the belt 164. In FIG. 3C, the holder 162 is illustrated in a cross-sectional view for convenience.

Returning to FIG. 2, the heating table 170 includes the holding holes 171 for holding the reaction containers 33 holding the samples, a heater 172 configured to heat the reaction containers 33 held in the respective holding holes 171, and the transfer unit 173 for transferring the reaction containers 33. The heating table 170, the holding holes 171, and the heater 172 correspond to the heating table 11, the holding holes 11a, and the heater 11b in FIG. 1, respectively.

The heating table 170 has a circular profile in a plan view and is configured to be rotatable in a circumferential direction. When the heating table 170 rotates in the circumferential direction, the holding holes 171 are moved in the circumferential direction. The heater 172 heats the reaction containers 33 held in the holding holes 171 to 37°C. The transfer unit 173 is configured to be rotatable in the circumferential direction of the heating table 170.

The reagent table 180 corresponds to the reagent table 23 in FIG. 1. The reagent table 180 is configured such that the reagent containers 34 holding reagents can be placed therein. In the reagent table 180, three reagent racks 181 each capable of holding ten reagent containers 34 are set in an outer peripheral portion and four reagent racks 182 each capable of holding two reagent containers 34 are set in an inner peripheral portion. The reagent table 180 is configured to be rotatable in a circumferential direction. The reagent table 180 rotates in the circumferential direction to transfer the reagent containers 34, placed in the reagent table 180 by using the reagent racks 181, 182, to a first aspirating position 241 at which the reagent dispensing unit 240 aspirates the reagent and to a second aspirating position 251 at which the reagent dispensing unit 250 aspirates the reagent.

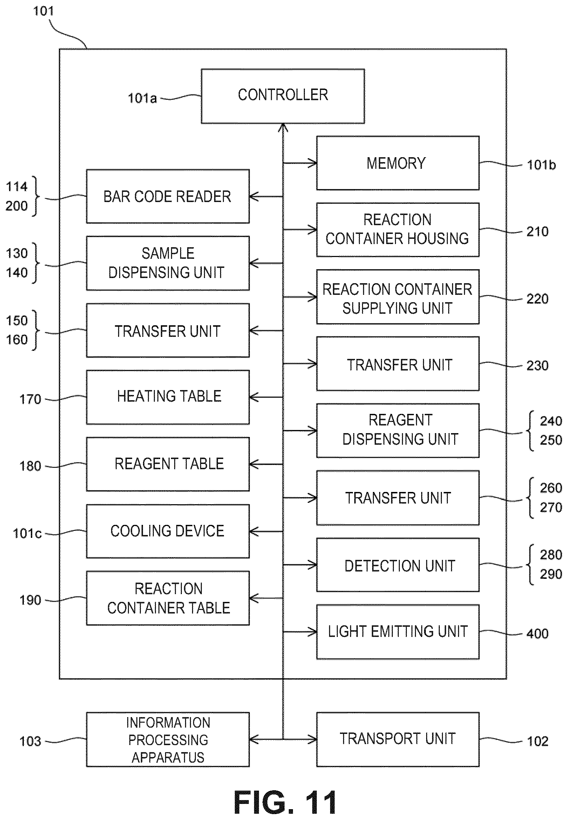

A cooling device 101c is installed below the reagent table 180 as illustrated in FIG. 11. The cooling device 101c cools the reagent containers 34 held in the reagent table 180 to a predetermined temperature. The cooling device 101c is configured by, for example, a Peltier device.

The reagent dispensing units 240, 250 correspond to the first reagent dispensing unit 12 and the second reagent dispensing unit 13 in FIG. 1, respectively. The reagent dispensing units 240, 250 dispense the reagents into the reaction containers 33 heated by the heating table 170.

For example, when the adjustment reagent is to be dispensed into the reaction container 33, the transfer unit 173 of the heating table 170 disposes the reaction container 33 held in the holding hole 171 of the heating table 170 at a first discharging position 261 or a second discharging position 271. The reagent dispensing unit 240 or the reagent dispensing unit 250 aspirates the adjustment reagent from the reagent container 34 disposed at the first aspirating position 241 or the second aspirating position 251, heats the aspirated adjustment reagent, and dispenses the heated reagent into the reaction container 33 disposed at the first discharging position 261 or the second discharging position 271. The adjustment reagent is thereby mixed with the sample. Thereafter, the transfer unit 173 sets the reaction container 33 in the holding hole 171 of the heating table 170 again.

When the trigger reagent is to be dispensed into the reaction container 33, the transfer unit 260 or the transfer unit 270 disposes the reaction container 33 held in the holding hole 171 of the heating table 170 at the first discharging position 261 or the second discharging position 271. The reagent dispensing unit 240 or the reagent dispensing unit 250 aspirates the trigger reagent from the reagent container 34 disposed at the first aspirating position 241 or the second aspirating position 251, heats the aspirated trigger reagent, and dispenses the heated reagent into the reaction container 33 disposed at the first discharging position 261 or the second discharging position 271. The trigger reagent is thereby mixed with the sample. Thereafter, the transfer unit 260 or the transfer unit 270 sets the reaction container 33 in a holder 281 of the detection unit 280 or a holder 291 of the detection unit 290.

As described above, in some cases, only the trigger reagent is discharged into the reaction container 33 and, in the other cases, the adjustment reagent is discharged into the reaction container 33 together with the trigger reagent, depending on the measurement item set for the sample. When the trigger agent is to be discharged into reaction container 33, the transfer unit 260 grips the reaction container 33 and disposes it at the first discharging position 261, or the transfer unit 270 grips the reaction container 33 and disposes it at the second discharging position 271. When the trigger reagent has been discharged, the transfer units 260, 270 transfer the reaction containers 33 to the detection units 280, 290, respectively. When the adjustment reagent is to be discharged into the reaction container 33, the transfer unit 173 grips the reaction container 33 and disposes it at the first discharging position 261 or the second discharging position 271. When the trigger agent has been discharged, the transfer unit 173 returns the reaction container 33 to the holding hole 171 of the heating table 170.

The transfer units 260, 270 correspond to the first transfer unit 16 and the second transfer unit 17 in FIG. 1, respectively.

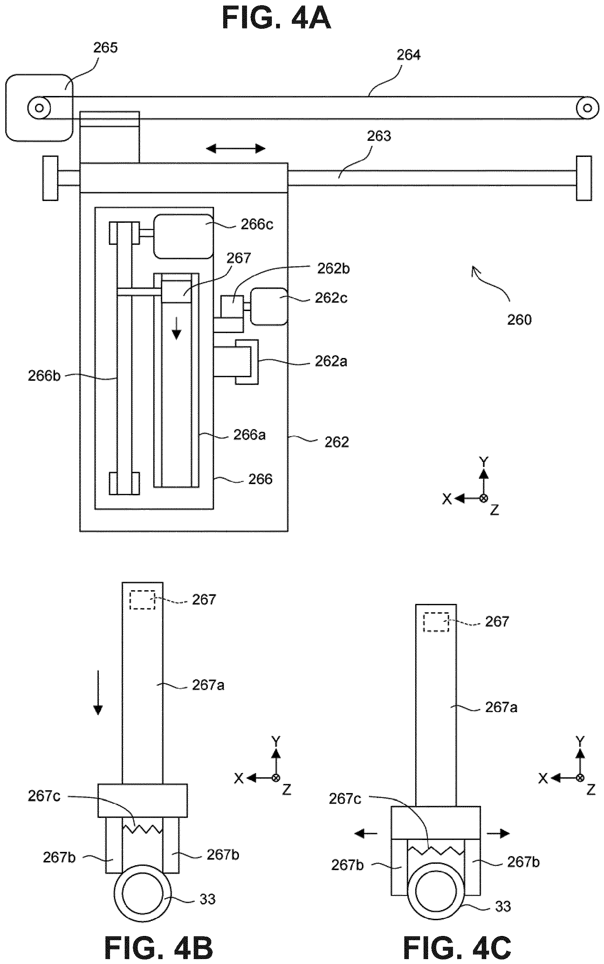

As illustrated in FIG. 4A, the transfer unit 260 includes a supporting member 262, a rail 263 configured to guide the supporting member 262, a belt 264 connected to the supporting member 262, and a motor 265 configured to drive the belt 264. The rail 263 is provided to extend in the X-axis direction. The belt 264 is arranged parallel to the rail 263. The belt 264 is wound around a drive shaft of the motor 265 and a pulley. An end portion of the supporting member 262 is attached to the belt 264. The motor 265 drives the belt 264 and the supporting member 262 is thereby transferred in the X-axis direction while being guided by the rail 263.

The supporting member 262 is provided with a supporting member 266, a rail 262a configured to guide the supporting member 266, a belt 262b connected to the supporting member 266, and a motor 262c configured to drive the belt 262b. The rail 262a is provided to extend in the Z-axis direction. The belt 262b is arranged parallel to the rail 262a. The belt 262b is wound around a drive shaft of the motor 262c and a pulley. An end portion of the supporting member 266 is attached to the belt 262b. The motor 262c drives the belt 262b and the supporting member 266 is thereby transferred in the Z-axis direction while being guided by the rail 262a.

The supporting member 266 is provided with a supporting member 267, a rail 266a configured to guide the supporting member 267, a belt 266b connected to the supporting member 267, and a motor 266c configured to drive the belt 266b. The rail 266a is provided to extend in the Y-axis direction. The belt 266b is arranged parallel to the rail 266a. The belt 266b is wound around a drive shaft of the motor 266c and a pulley. An end portion of the supporting member 267 is attached to the belt 266b. The motor 266c drives the belt 266b and the supporting member 267 is thereby transferred in the Y-axis direction while being guided by the rail 266a.

As illustrated in FIG. 4B, a Y-axis positive side end portion of an arm 267a is provided on a Z-axis negative side surface of the supporting member 267. Accordingly, the arm 267a moves in the Y-axis direction with the movement of the supporting member 267. Paired claws 267b are provided in a Y-axis negative side end portion of the arm 267a to be movable toward and away from each other in the X-axis direction. A spring 267c is laid between the paired claws 267b. The spring 267c biases the paired claws 267b in the directions toward each other. As illustrated in FIG. 4B, the movement of the paired claws 267b is restricted at positions at a predetermined interval and the paired claws 267b are disposed at these positions.

When the motor 266c is driven to move the supporting member 267 in the Y-axis negative direction, the arm 267a is moved in the Y-axis negative direction. When the arm 267a is moved further in the Y-axis negative direction from a state where the paired claws 267b are in contact with a side surface of the reaction container 33 as illustrated in FIG. 4B, the claws 267b slide on the side surface of the reaction container 33 and open in the directions away from each other. As illustrated in FIG. 4C, the paired claws 267b thereby grip the reaction container 33. The spring 267c applies force for gripping the reaction container 33 to the paired claws 267b. The paired claws 267b form a gripper configured to grip the reaction container 33.

The transfer unit 260 drives the motors 265, 262c, 266c as described above to move the claws 267b in the X-axis, Y-axis, and Z-axis directions. The transfer unit 260 thus grips and transfers the reaction container 33. Grip on the reaction container 33 is released, for example, by moving the claws 272b in the Y-axis positive direction with the reaction container 33 inserted in the holder 281. The claws 267b thereby slide on the side surface of the reaction container 33 and the grip on the reaction container 33 is released.

The transfer unit 270 also has a configuration similar to the transfer unit 260. The transfer unit 173 is has a modified configuration in which the rail 263 and the belt 264 rotate the supporting member 262. For example, the supporting member 262 is rotatably supported on a supporting shaft and a transmission mechanism such as a gear transmits drive force of the motor 265 to the supporting member 262. Other configurations of the transfer unit 173 are substantially the same as those of the transfer unit 260.

The transfer unit 260 transfers the reaction container 33 held in the holding hole 171 of the heating table 170 to the first discharging position 261 when the trigger reagent is to be discharged into the reaction container 33. After the trigger reagent is discharged into the reaction container 33, the transfer unit 260 transfers the reaction container 33 to the holder 281 in the detection unit 280. The transfer unit 270 transfers the reaction container 33 held in the holding hole 171 of the heating table 170 to the second discharging position 271 when the trigger reagent is to be discharged into the reaction container 33. After the trigger reagent is discharged into the reaction container 33, the transfer unit 270 transfers the reaction container 33 to the holder 291 in the detection unit 290.

As described above, the reagent dispensing unit 240 dispenses the trigger reagent into the reaction container 33 at the first discharging position 261 on a path in which the transfer unit 260 transfers the reaction container 33. The reagent dispensing unit 250 dispenses the trigger reagent into the reaction container 33 at the second discharging position 271 on a path in which the transfer unit 270 transfers the reaction container 33. The reaction containers 33 can be thereby promptly transferred to the detection units 280, 290 after the dispensing of the trigger reagent. Moreover, since the reagent dispensing units 240, 250 dispense the trigger reagent, the trigger reagent can be dispensed independently into the reaction containers 33 transferred by the transfer units 260, 270. This can reduce waiting time for dispensing the trigger reagent into the reaction containers 33 and thereby improve processing efficiency of the sample analyzer 100.

The detection unit 280 includes the holders 281 and detectors 282 provided to correspond to the respective holders 281. The detection unit 290 includes the holders 291 and detectors 292 provided to correspond to the respective holders 291. The detection units 280, 290 correspond to the first detection unit 14 and the second detection unit 15 in FIG. 1, respectively. The holders 281, 291 correspond to the first holders 14a and the second holders 15a in FIG. 1, respectively. The detectors 282, 292 correspond to the first detectors 14b and the second detectors 15b in FIG. 1, respectively. The holders 281 hold the reaction containers 33 each holding the measurement specimen containing the sample and the reagent dispensed by the reagent dispensing unit 240. The holders 291 hold the reaction containers 33 each holding the measurement specimen containing the sample and the reagent dispensed by the reagent dispensing unit 250. The detectors 282, 292 detect signals for analysis obtained from the measurement specimens in the reaction containers 33 held by the holders 281, 291.

The information processing apparatus 103 includes a controller 103a. The controller 103a corresponds to the second controller 26b in FIG. 1. The controller 103a analyzes the samples in the reaction containers 33 held by the holders 281 based on the signals detected by the detectors 282 and analyzes the samples in the reaction containers 33 held by the holders 291 based on the signals detected by the detectors 292.

The discarding unit 303 corresponds to the discarding unit 24 in FIG. 1. The discarding unit 303 includes the disposal openings 301, 302. The transfer unit 260 transfers the reaction containers 33 which are held by the holders 281 of the detection unit 280 and which are to be discarded to the discarding unit 303 by transferring the reaction containers 33 to the disposal opening 301. The transfer unit 270 transfers the reaction containers 33 which are held by the holders 291 of the detection unit 290 and which are to be discarded to the discarding unit 303 by transferring the reaction containers 33 to the disposal opening 302.

The transfer unit 260 has the function of the first transfer unit 16 and the function of the first discarding transfer unit 18 in FIG. 1. The transfer unit 270 has the function of the second transfer unit 17 and the function of the second discarding transfer unit 19 in FIG. 1. When the transfer unit 260 achieves the functions of the first transfer unit 16 and the first discarding transfer unit 18 and the transfer unit 270 achieves the functions of the second transfer unit 17 and the second discarding transfer unit 19 as described above, the configuration of the sample analyzer 10 can be simplified.

The number of disposal openings communicating with the discarding unit 303 may be one. Also, in this case, the transfer units 260, 270 transfer the reaction containers 33 to be discarded to the one disposal opening. Moreover, two discarding units may be provided to correspond to the disposal openings 301, 302, respectively.

The reaction container table 190 has a ring shape in the plan view and is arranged outside the reagent table 180. The reaction container table 190 is configured to be rotatable in the circumferential direction. The reaction container table 190 includes holding holes for holding the reaction containers 33. The bar code reader 200 reads bar codes attached to the reagent racks 181, 182 and the bar codes attached to the reagent containers 34. The bar codes of the reagent racks 181, 182 hold identification information for identifying the reagent racks 181, 182 and the bar codes of the reagent containers 34 hold identification information for identifying the reagent containers 34.

The reaction container housing unit 210 houses the new reaction containers 33. The reaction container supplying unit 220 supplies the reaction containers 33 from the reaction container housing unit 210.

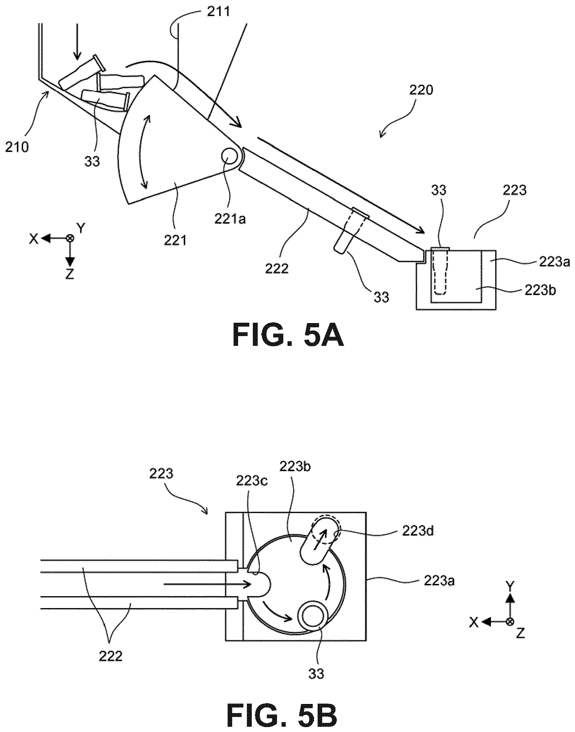

As illustrated in FIG. 5A, the reaction container housing unit 210 includes an introduction opening 211 into which the user can introduce the new reaction containers 33, and houses the reaction containers 33 introduced from the introduction opening 211. The reaction container supplying unit 220 includes a take-out mechanism 221, a guide 222, and a send-out mechanism 223. The take-out mechanism 221 takes out the reaction containers 33 from the reaction container housing unit 210 one by one. The guide 222 is configured by two rails which support a bottom surface of the flange 33b of each reaction container 33. Each reaction container 33 taken out by the take-out mechanism 221 slides down along the guide 222 with the bottom surface of the flange 33b supported by the guide 222 and is transferred to the send-out mechanism 223.

As illustrated in FIG. 5B, the send-out mechanism 223 includes a supporting stage 223a and a rotary table 223b. Notches 223c provided in the rotary table 223b hold the reaction containers 33 transferred by the guide 222. The rotary table 223b rotates to transfer the reaction containers 33 held in the notches 223c. A notch 223d provided in the supporting stage 223a holds one of the reaction containers 33 transferred by the rotary table 223b.

Returning to FIG. 2, the transfer unit 230 transfers the new reaction container 33 held in the notch 223d of the reaction container supplying unit 220 to the holding holes 152a of the transfer unit 150 and to the holding holes of the reaction container table 190. Moreover, the transfer unit 260 transfers the new reaction container 33 held in the notch 223d of the reaction container supplying unit 220 to the holding holes 162a of the transfer unit 160. As described above, the new reaction container 33 supplied by the reaction container supplying unit 220 is transferred to the transfer units 150, 160 and the reaction container table 190. This can suppress an increase in the installation area of the sample analyzer 100 compared to the case where multiple reaction container supplying units are provided to supply the new reaction containers 33 to the transfer units 150, 160 and the reaction container table 190.

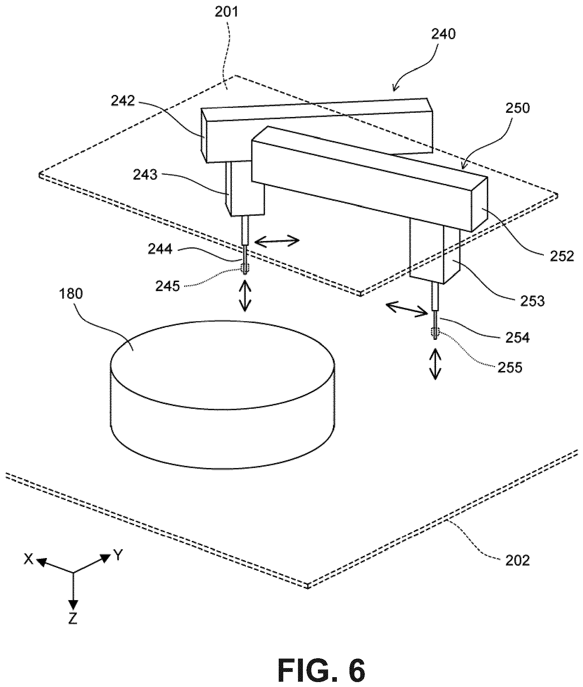

As illustrated in FIG. 6, the reagent dispensing units 240, 250 are supported on a support unit 201 arranged above the reagent table 180. The reagent table 180 is installed on a base 202. The positions of the reagent dispensing units 240, 250 in an X-Y plane and the position of the reagent table 180 in an X-Y plane partially overlap each other. In this configuration, there is no need to provide a space for installing the reagent dispensing units 240, 250 in the base 202. This can suppress an increase in the installation area of the sample analyzer 100 compared to the case where the reagent dispensing units 240, 250 are arranged on the base 202.

The reagent dispensing unit 240 includes a horizontal transfer unit 242, a vertical transfer unit 243, a first pipet 244, a first heater 245, and a not-illustrated pump for aspirating and discharging the reagent by using the first pipet 244. The horizontal transfer unit 242 transfers the vertical transfer unit 243 in a predetermined direction in a horizontal plane, that is, an X-Y plane. The vertical transfer unit 243 transfers the first pipet 244 in the vertical direction, that is the Z-axis direction. The first heater 245 is installed near a lower end of the first pipet 244 and heats the reagent held the first pipet 244.

When dispensing the reagent, the reagent dispensing unit 240 inserts the first pipet 244 into the reagent container 34 disposed at the first aspirating position 241 illustrated in FIG. 2 and aspirates the reagent in the reagent container 34. The reagent dispensing unit 240 heats the reagent held in the first pipet 244 to a predetermined temperature by using the first heater 245. Thereafter, the reagent dispensing unit 240 discharges the reagent held in the first pipet 244 into the reaction container 33 disposed at the first discharging position 261.

Similarly, the reagent dispensing unit 250 includes a horizontal transfer unit 252, a vertical transfer unit 253, a second pipet 254, a second heater 255, and a not-illustrated pump for aspirating and discharging the reagent by using the second pipet 254. The horizontal transfer unit 252 transfers the vertical transfer unit 253 in a predetermined direction in a horizontal plane, that is, an X-Y plane. The vertical transfer unit 253 transfers the second pipet 254 in the vertical direction, that is the Z-axis direction. The second heater 255 is installed near a lower end of the second pipet 254 and heats the reagent held the second pipet 254.

When dispensing the reagent, the reagent dispensing unit 250 inserts the second pipet 254 into the reagent container 34 disposed at the second aspirating position 251 illustrated in FIG. 2 and aspirates the reagent in the reagent container 34. The reagent dispensing unit 250 heats the reagent held in the second pipet 254 to a predetermined temperature by using the second heater 255. Thereafter, the reagent dispensing unit 250 discharges the reagent held in the second pipet 254 into the reaction container 33 disposed at the second discharging position 271.

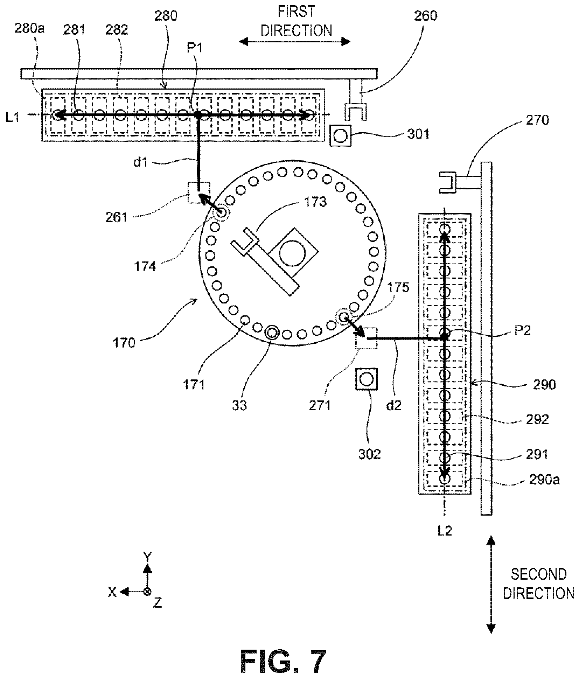

As illustrated in FIG. 7, in the detection unit 280, the holders 281 are arranged side by side or aligned in a region 280a in a first direction, that is the X-axis direction. In the detection unit 290, the holders 291 are arranged side by side or aligned in a region 290a in a second direction, that is the Y-axis direction. The heating table 170 is arranged between the row of the holders 281 of the detection unit 280 and the row of the holders 291 of the detection unit 290. By arranging the detection units 280, 290 and the heating table 170 as described above, the detection units 280, 290 and the heating table 170 can be collectively arranged in a compact manner. This can suppress an increase in the installation area of the sample analyzer 100.

When the reagent dispensing unit 240 is to discharge the reagent into the reaction container 33 held in the holding hole 171 of the heating table 170, the transfer unit 173 or the transfer unit 260 takes out the reaction container 33 from the holding hole 171 at a first position 174 and disposes the reaction container 33 at the first discharging position 261. The transfer unit 260 transfers the reaction container 33 into which the trigger reagent is discharged at the first discharging position 261, in the Y-axis positive direction from the first discharging position 261 to dispose the reaction container 33 in the detection unit 280. Thereafter, the transfer unit 260 transfers the reaction container 33 in the X-axis direction to transfer the reaction container 33 to one of the holders 281 of the detection unit 280. The transfer unit 260 transfers the reaction container 33 in the Y-axis positive direction to an overhead position P1 directly above the row L1 of the holders 281 of the detection unit 280 and then transfers the reaction container 33 such that time it takes to transfer the reaction container 33 to any holder 281 is predetermined time irrespective of the position of the holder 281. The distance by which the transfer unit 260 transfers the reaction container 33 from the first discharging position 261 to the overhead position P1 is referred to as d1.

Similarly, when the reagent dispensing unit 250 is to discharge the reagent into the reaction container 33 held in the holding hole 171 of the heating table 170, the transfer unit 173 or the transfer unit 270 takes out the reaction container 33 from the holding hole 171 at a second position 175 and disposes the reaction container 33 at the second discharging position 271. The transfer unit 270 transfers the reaction container 33 into which the trigger reagent is discharged at the second discharging position 271, in the X-axis negative direction from the second discharging position 271 to dispose the reaction container 33 in the detection unit 290. Thereafter, the transfer unit 270 transfers the reaction container 33 in the Y-axis direction to transfer the reaction container 33 to one of the holders 291 of the detection unit 290. The transfer unit 270 transfers the reaction container 33 in the X-axis negative direction to an overhead position P2 directly above the row L2 of the holders 291 of the detection unit 290 and then transfers the reaction container 33 such that time it takes to transfer the reaction container 33 to any holder 291 is predetermined time irrespective of the position of the holder 291. The distance by which the transfer unit 270 transfers the reaction container 33 from the second discharging position 271 to the overhead position P2 is referred to as d2.

Here, the distance by which the transfer unit 260 transfers the reaction container 33 from the first discharging position 261 to the detection unit 280 is substantially equal to the distance by which the transfer unit 270 transfers the reaction container 33 from the second discharging position 271 to the detection unit 290. Specifically, the first discharging position 261, the second discharging position 271, and the positions of the detection units 280, 290 are set such that d1 = d2 is satisfied. Moreover, the positional relationship of the overhead position P1 to the detection unit 280 is substantially the same as the positional relationship of the overhead position P2 to the detection unit 290, and the pitch of the holders 281 is substantially the same as the pitch of the holders 291. The distance from the first discharging position 261 to each holder 281 of the detection unit 280 is thus substantially equal to the distance from the second discharging position 271 to the holder 291 of the detection unit 290 whose positional relationship corresponds to that of the holder 281.

As described above, the transfer distance from the position where the trigger reagent is dispensed to each holder 281 in the detection unit 280 in the case where the reagent dispensing unit 240 dispenses the trigger reagent is substantially equals to the transfer distance from the position where the trigger reagent is dispensed to the holder 291 corresponding to the holder 281 in the detection unit 290 in the case where the reagent dispensing unit 250 dispenses the trigger reagent. This can make the time from the dispensing of the trigger reagent to the start of the detection in the case where the measurement is performed in the detector 282 of the detection unit 280 substantially the same as that in the case where the measurement is performed in the detector 292 of the detection unit 290, with substantially the same transfer control applied to the detection unit 280 and the detection unit 290. This can suppress differences in the measurement results obtained by the detection units 280, 290.

The overhead position P1 is preferably arranged at a center position of the region 280a in the X-axis direction, that is the alignment direction of the holders 281. This can reduce the distance between the overhead position P1 and the holder 281 farthest from the overhead position P1. The transfer time can be thereby reduced when the transfer time is adjusted such that the reaction container 33 is transferred from the first discharging position 261 to each holder 281 in the same time. Similarly, the overhead position P2 is preferably arranged at a center position of the region 290a in the Y-axis direction, that is the alignment direction of the holders 291.

Although the transfer paths of the transfer units 260, 270 are straight lines, the transfer paths of the transfer units 260, 270 may include curves. In this case, the distances transferred by the transfer units 260, 270 are the transfer distances of the reaction containers 33 transferred along curved and straight transfer paths.

Although the transfer distances d1, d2 are set to be equal, the transfer distances d1, d2 do not have to be equal. The transfer distances d1, d2 only have to be set such that the differences in measurement results caused by the difference between the transfer distances d1, d2 is a degree which causes no clinical differences in the results of analysis performed by the information processing apparatus 103.

The transfer unit 260 transfers the reaction container 33 held in the holding hole 171 disposed at the first position 174 to the holder 281. The transfer unit 270 transfers the reaction container 33 held in the holding hole 171 disposed at the second position 175 different from the first position 174 to the holder 291. The transfer units 260, 270 can thereby transfer the reaction containers 33 held in the heating table 170 from the two different positions to the detection units 280, 290, respectively, in parallel.

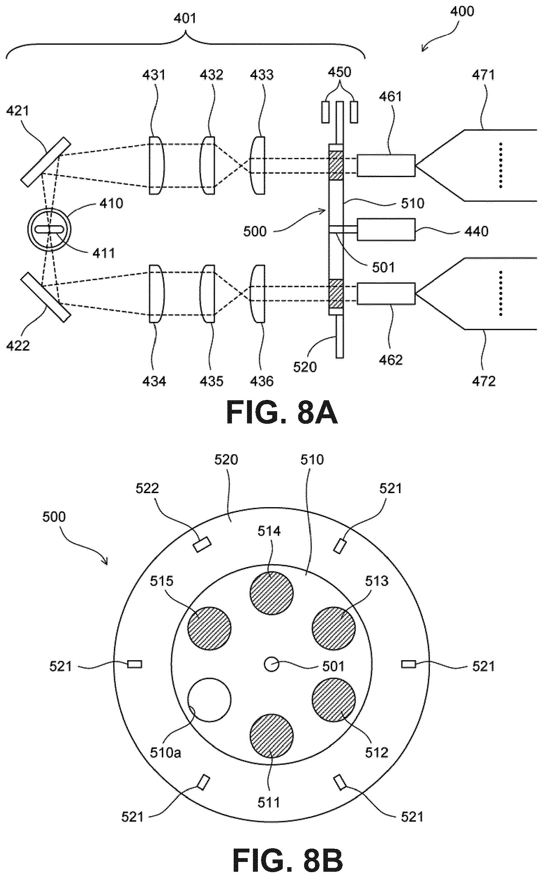

As illustrated in FIG. 8A, a light emitting unit 400 includes a light source unit 401, bundle members 461, 462, 13 first optical fibers 471, and 13 second optical fibers 472. The light source unit 401 includes a light source 410, mirrors 421, 422, condenser lenses 431 to 436, a motor 440, a light-transmissive sensor 450, a disc-shaped filter portion 500.

The light source 410 is configured by a halogen lamp. The light source 410 includes a plate-shape filament 411 which emits light from both sides and light of the same properties are emitted from both sides of the filament 411. The light of the same properties is thereby emitted from the light source 410 toward each of the mirrors 421, 422. The mirrors 421, 422 reflect the light emitted from the light source 410.

The condenser lenses 431 to 433 focus the light reflected by the mirror 421. The light focused by the condenser lenses 431 to 433 transmits through one of optical filters 511 to 515 of the filter portion 500 and is guided to the first optical fibers 471. The condenser lenses 434 to 436 focus the light reflected by the mirror 422. The light focused by the condenser lenses 434 to 436 transmits through one of optical filters 511 to 515 of the filter portion 500 and is guided to the second optical fibers 472. The filter portion 500 is rotatable about a shaft 501 and the shaft 501 is connected to a rotary shaft of the motor 440.

The 13 first optical fibers 471 are bundled with the bundle member 461 and the 13 second optical fibers 472 are bundled with the bundle member 462. Front ends of the first optical fibers 471 are connected to the detection unit 280. Front ends of the second optical fibers 472 are connected to the detection unit 290. The first optical fibers 471 guide the light emitted from the light source unit 401 to the detectors 282 of the detection unit 280 and the second optical fibers 472 guide the light emitted from the light source unit 401 to the detectors 292 of the detection unit 290.

Since the light emitted from one light source unit 401 is guided to the detectors 282, 292 as described above, the measurement results based on the detectors 282, 292 are less likely to vary.

As illustrated in FIG. 8B, the filter portion 500 include a filter plate 510 and a holding member 520. Six holes 510a are formed in the filter plate 510 on the same circumference at intervals of 60°, and the optical filters 511 to 515 are fitted to five of the six holes 510a. Each of the optical filters 511 to 515 is a band-pass filter which transmits light having a predetermined wavelength band and block light having other wavelength bands. Center wavelengths of transmittance wavelength bands of the optical filters 511 to 515 are 340 nm, 405 nm, 575 nm, 660 nm, and 800 nm, respectively. The hole 510a fitted with no optical filter is closed so as not to transmit light. The light with a wavelength of 660 nm is used for blood coagulation time measurement, the light with a wavelength of 405 nm is used for synthetic substrate measurement, and the light with a wavelength of 800 nm is used for immunonephelometry measurement.

The holding member 520 holds the filter plate 510 such that both sides of the optical filters 511 to 515 are exposed. The filter plate 510 is fixed to the holding member 520. One slit 521 and five slits 522 are formed in the holding member 520 on the same circumference at intervals of 60°. The slit 521 has a larger width in the rotating direction than the slits 522.

When the filter portion 500 rotates, the optical filters 511 to 515 are sequentially arranged in a passage of light focused by the condenser lenses 431 to 433 and in a passage of light focused by the condenser lenses 434 to 436. Moreover, when the filter portion 500 rotates, the slits 521, 522 pass a detection position of the sensor 450. The rotating position of the filter portion 500 is thus recognized by using the detection signals of the sensor 450. The light having transmitted through one of the optical filters 511 to 515 enters end portions of the first optical fibers 471 bundled by the bundle member 461 and end portions of the second optical fibers 472 bundled by the bundle member 462.

The rotation of the filter portion 500 is controlled such that the angular velocity thereof is constant. Light having different wavelength bands is thereby supplied to the first optical fibers 471 and the second optical fibers 472 at constant time intervals. A detection signal corresponding to the slit 521 out of the detection signals of the sensor 450 is used for the rotation control of the filter portion 500. Specifically, the motor 440 is controlled such that the detection signal corresponding to the slit 521 is periodically detected. Moreover, detection signals corresponding to the slits 522 out of the detection signals of the sensor 450 are used to determine light having which wavelength band is supplied to each of the first optical fibers 471 and the second optical fibers 472. Specifically, the wavelength band of the supplied light is identified by determining the number of detection signals corresponding to the slits 522 which are detected from the detection of the detection signal corresponding to the slit 521. In the measurement, the filter portion 500 rotates at a speed of, for example, about 10 revolutions per second.

The light source unit 401 thus emits the light with the wavelength of 660 nm for the blood coagulation time measurement, the light with the wavelength of 405 nm for the synthetic substrate measurement, and the light with the wavelength of 800 nm for the immunonephelometry measurement repeatedly in order.

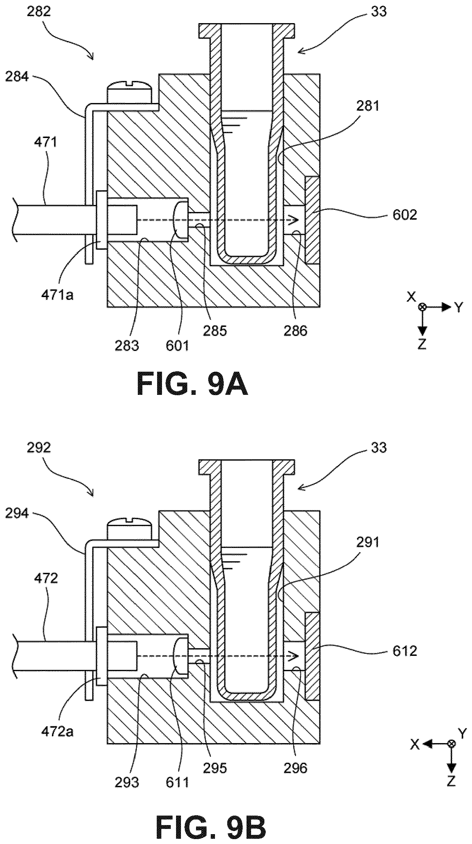

As illustrated in FIG. 9A, the detector 282 includes a condenser lens 601 and a sensor 602. An end portion 471a of the first optical fiber 471 is inserted into a circular hole 283 and a leaf spring 284 presses a back surface of the end portion 471 a. The end portion 471a is thereby fixed to the hole 283. The condenser lens 601 is attached to a Y-axis positive side surface of the hole 283. A hole 285 allows the hole 283 and the holder 281 to communicate with each other. The light outputted from the end portion 471a and focused by the condenser lens 601 passes the hole 285 and is guided to the reaction container 33 held by the holder 281. The condenser lens 601 forms a light irradiation portion which irradiate the reaction container 33 with the light outputted from the first optical fiber 471.

A hole 286 allows the sensor 602 and the holder 281 to communicate with each other. The light guided from the condenser lens 601 to the reaction container 33 transmits through the reaction container 33 and the measurement specimen and is then guided to the sensor 602. The sensor 602 receives the light from the reaction container 33 held by the holder 281 and outputs the signals for analysis. The detector 282 may include optical elements other than the condenser lens 601 such as a collimator lens in the configuration of the light irradiation portion.

As illustrated in FIG. 9B, the detector 292 includes a condenser lens 611 and a sensor 612 like the detector 282. An end portion 472a of the second optical fiber 472 is inserted into a circular hole 293 and a leaf spring 294 presses a back surface of the end portion 472a. The end portion 472a is thereby fixed to the hole 293. The condenser lens 611 is attached to an X-axis negative side surface of the hole 293. A hole 295 allows the hole 293 and the holder 291 to communicate with each other. The light outputted from the end portion 472a and focused by the condenser lens 611 passes the hole 295 and is guided to the reaction container 33 held by the holder 291. The condenser lens 611 forms a light irradiation portion which irradiate the reaction container 33 with the light outputted from the second optical fiber 472.

A hole 296 allows the sensor 612 and the holder 291 to communicate with each other. The light guided from the condenser lens 611 to the reaction container 33 transmits through the reaction container 33 and the measurement specimen and is then guided to the sensor 612. The sensor 612 receives the light from the reaction container 33 held by the holder 291 and outputs the signals for analysis. The detector 292 may include optical elements other than the condenser lens 611 such as a collimator lens in the configuration of the light irradiation portion.

The signals outputted from the sensors 602 of the detectors 282 and the signals outputted from the sensors 612 of the detectors 292 are sent to the controller 103a of the information processing apparatus 103 in FIG. 2. The controller 103a analyze the samples in the reaction containers 33 held by the holders 281 based on changes with time in the signals outputted from the sensors 602. The controller 103a analyze the samples in the reaction containers 33 held by the holders 291 based on changes with time in the signals outputted from the sensors 612.

For example, in the analysis based on the coagulation time method, the controller 103a calculates the absorbance of each of the measurement specimens based on the detection signal outputted from the corresponding one of the sensors 602, 612 and calculates the time it takes for the calculated absorbance to exceed a predetermined threshold, as the coagulation time of the measurement specimen. The controller 103a may obtain turbidity instead of the absorbance from the detection signal and calculate the time it takes for the turbidity to exceed a predetermined threshold, as the coagulation time of the sample. Moreover, the controller 103a may calculate the time it takes for the detection signal outputted from each of the sensors 602, 612 to exceed a predetermined threshold as the coagulation time of the measurement specimen.

Although the configurations of the detectors 282, 292 in the case of detecting the light transmitted through the measurement specimens are illustrated in FIGS. 9A and 9B, the configurations may be such that the sensors 602, 612 receive light scattered by the measurement specimens and the controller 103a performs analysis by using any of the aforementioned methods based on the detection signals based on the scattered lights. In this case, in each detector 282, the arrangement of the sensor 602 and the hole 286 is modified and, in each detector 292, the arrangement of the sensor 612 and the hole 296 is modified.

Signals outputted from the sensors 602, 612 include signals based on light having all wavelengths obtained by irradiating the measurement specimens with light having all wavelengths. The signals based on light having all wavelengths are transmitted to the information processing apparatus 103. The controller 103a of the information processing apparatus 103 analyzes each of the samples by using a signal based on light having a wavelength corresponding to the measurement item set for the sample out of the received signals based on light having all wavelengths. Specifically, the controller 103a generates time-series data for each of the aforementioned five light wavelengths and analyzes the sample by using data corresponding to the measurement item of the sample out of the generated time-series data. High processing capability can be thereby maintained for any measurement item.

The coagulation time of each measurement specimen is calculated based on optical information such as the absorbance and the turbidity obtained from the measurement specimen as described above. Activated partial thromboplastin time, prothrombin time, and the like can be given as examples of "coagulation time."

Moreover, the coagulation time may be measured based on information other than the optical information such as an increase in viscosity due to coagulation of blood. When the coagulation time is calculated based on the increase in viscosity, the detectors 282, 292 each include a high-frequency wave transmission coil, a high-frequency wave reception coil, a reaction container placing portion which is provided between the high-frequency wave transmission coil and the high-frequency wave reception coil and in which the reaction container holding a steel ball is placed, and electromagnets which are provided on both ends of the reaction container placing portion. The steel ball in the reaction container oscillates to left and right by the magnetic force generated by the electromagnets. This oscillation decreases as the viscosity increases. When the coagulation of the measurement specimen starts, the viscosity of the measurement specimen increases and thus the oscillation of the steel ball decreases. Accordingly, the detectors 282, 292 detect changes in the oscillation by using the high-frequency wave reception coil to receive a high-frequency wave transmitted by the high-frequency wave transmission coil. The controller 103a of the information processing apparatus 103 calculates the coagulation time based on the changes in the detected oscillation over time.

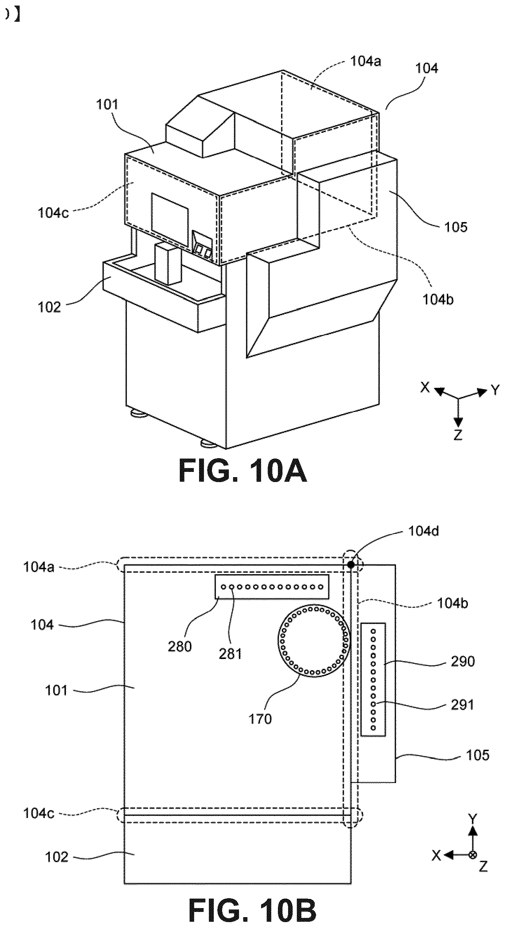

As illustrated in FIG. 10A, the sample analyzer 100 includes housings 104, 105. The housing 104 covers units of the measurement unit 101 located on the X-axis positive side of the transfer unit 160. The housing 104 includes a first side surface 104a which is located on the Y-axis positive side and which is substantially parallel to an X-Z plane, a second side surface 104b which is located on the X-axis negative side and which is substantially parallel to a Y-Z plane, and a third side surface 104c which is located on the Y-axis negative side and which is substantially parallel to the X-Z plane. The second side surface 104b is adjacent to or abuts on the first side surface 104a. The third side surface 104c is not adjacent to or does not abut on the first side surface 104a but is adjacent to or abuts on the second side surface 104b. The housing 105 covers the transfer unit 160, the transfer unit 270, and detection unit 290. The housing 105 is provided on the housing 104 so as to partially cover the second side surface 104b.

As illustrated in FIG. 10B, the first side surface 104a and the second side surface 104b are adjacent to or abut on each other in an adjacent part 104d. The adjacent part 104d extends in the Z-axis direction. The heating table 170 is arranged near the adjacent part 104d inside the housing 104. The detection unit 280 is arranged near one of the sides of the first side surface 104a close to the adjacent part 104d and extends along the first side surface 104a. The detection unit 290 is arranged near one of the sides of the second side surface 104b close to the adjacent part 104d and extends along the second side surface 104b. The holders 281 of the detection unit 280 are arranged side by side or aligned along the first side surface 104a in the plan view. The holders 291 of the detection unit 290 are arranged side by side or aligned along the second side surface 104b in the plan view. The transporter 102 is arranged along the third side surface 104c.

As illustrated in FIGS. 10A and 10B, since the heating table 170 and the detection units 280, 290 are arranged near the adjacent part 104d, the heating table 170 and the detection units 280, 290 can be provided close to one another in the measurement unit 101. This can suppress an increase in the installation area of the sample analyzer 100.

Moreover, the transporter 102 is arranged along the third side surface 104c and the detection units 280, 290 are arranged on the first side surface 104a side. Accordingly, the step of aspirating the samples from the sample containers 32 is performed in a front portion of the measurement unit 101 and the step of measuring the measurement specimens is performed in a rear portion of the measurement unit 101. Accordingly, it is only necessary to transfer the samples in one direction, that is from front to rear in the measurement unit 101, and the configuration of the measurement unit 101 can be thereby simplified.

As illustrated in FIG. 11, the measurement unit 101 includes the bar code readers 114, 200, the sample dispensing units 130, 140, the transfer units 150, 160, the heating table 170, the reagent table 180, the reaction container table 190, the reaction container housing unit 210, the reaction container supplying unit 220, the transfer unit 230, the reagent dispensing units 240, 250, the transfer units 260, 270, the detection units 280, 290, and the light emitting unit 400 as the configurations of a circuit portion as described with reference to FIG. 2.

The measurement unit 101 also includes a controller 101a, a memory 101b, and the cooling device 101c as the configurations of the circuit portion. The controller 101 a includes an arithmetic processing unit such as a CPU and controls the units in the measurement unit 101 and the transporter 102 according to a program stored in the memory 101b. The memory 101b includes a recording medium such as a ROM, a RAM, and a hard disk drive, and stores the program and information necessary for the control of the controller 101a. The memory 101b is also utilized as a work region in the control.

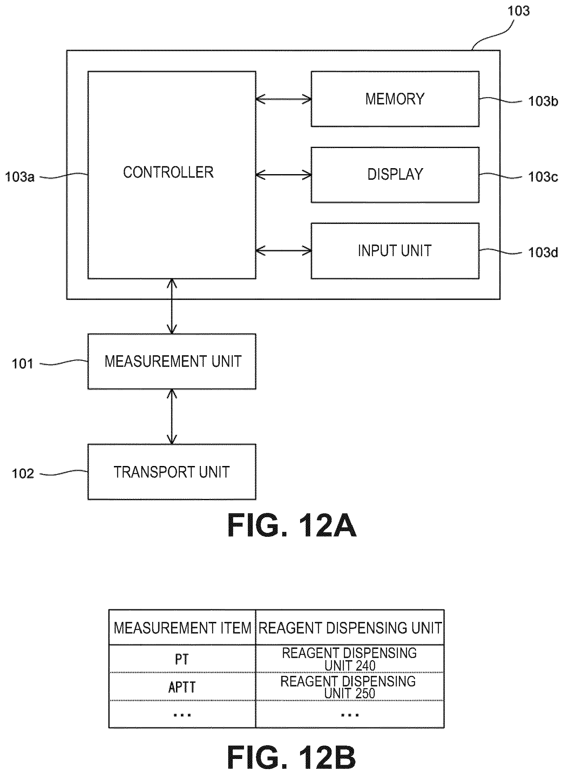

As illustrated in FIG. 12A, the information processing apparatus 103 includes the controller 103a, a memory 103b, a display 103c, and an input unit 103d. The controller 103a includes an arithmetic processing unit such as a CPU and performs analysis processing and controls the units in the information processing apparatus 103 according to a program stored in the memory 103b. The memory 103b includes a recording medium such as a ROM, a RAM, and a hard disk drive, and stores the program and information necessary for the processing and control of the controller 103a. The memory 103b is also utilized as a work region in the processing and control. The display 103c includes display means such as a monitor. The input unit 103d includes input means such as a keyboard and a mouse. The information processing apparatus 103 is configured by, for example, a personal computer.

The controller 101a of the measurement unit 101 outputs the detection signals outputted from the sensor 602, 612 as described above, to the controller 103a of the information processing apparatus 103. The controller 103a analyzes the measurement specimens based on the received detection signals. Specifically, the controller 103a calculates the absorbance based on the received detection signals as described above and calculates the coagulation time from the absorbance. The controller 103a displays the calculated coagulation time and the like on the display 103c as the analysis results.

As illustrated in FIG. 12B, the association information in which the measurement items and the reagent dispensing units to be used are associated with one another is stored in the memory 101b of the measurement unit 101 and the memory 103b of the information processing apparatus 103. When the controller 103a of the information processing apparatus 103 receives setting of the association information from the user via the input unit 103d, the controller 103a stores the received association information in the memory 103b and also sends the association information to the measurement unit 101. When the controller 101a of the measurement unit 101 receives the association information from the information processing apparatus 103, the controller 101a stores the received association information in the memory 101b.

By storing the association information in the memory 101b as described above, the association between the measurement items and the reagent dispensing units to be used in the measurement of the measurement items is set in the sample analyzer 100. When the measurement is to be performed according to the measurement items, the controller 101a of the measurement unit 101 determines the reagent dispensing unit to be used based on the association information stored in the memory 101b.Note that the memory 101b and the memory 103b form the memory 26c in FIG. 1.

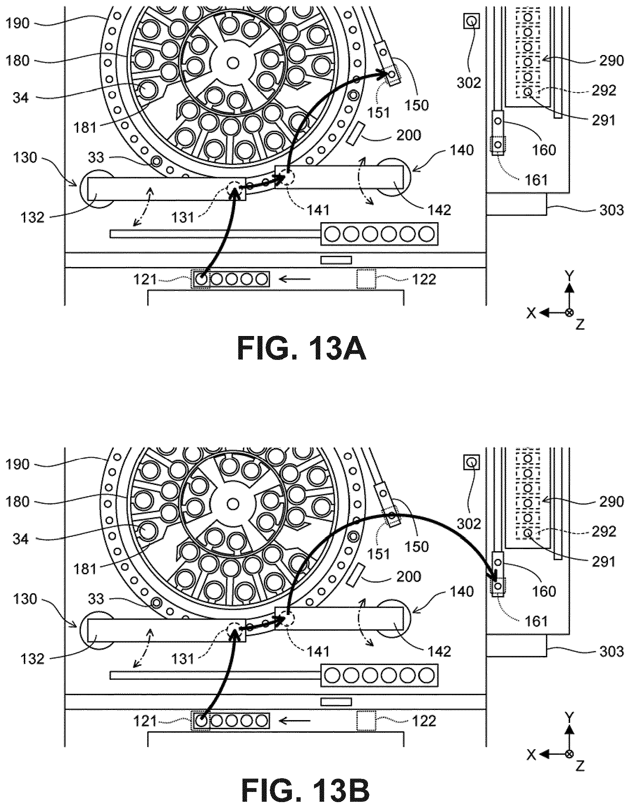

Next, the transfer paths of the sample and the reaction container 33 are described with reference to FIG. 13A to FIG. 15B.

Modes of aspirating the samples by the measurement unit 101 include a first aspirating mode and a second aspirating mode. When the aspirating mode set for the sample is the first aspirating mode, as illustrated in FIG. 13A, the sample dispensing unit 130 aspirates the sample from the sample container 32 at the sample aspirating position 121 and dispenses the aspirated sample into the reaction container 33 on the reaction container table 190. The reaction container table 190 is rotated to such a position that the sample dispensing unit 140 can aspirate the sample from the reaction container 33. The sample dispensing unit 140 aspirates the sample from the reaction container 33 on the reaction container table 190 and dispenses the aspirated sample into the reaction container 33 at the first sample discharging position 151 on the transfer unit 150. Alternatively, as illustrated in FIG. 13B, the sample dispensing unit 140 aspirates the sample from the reaction container 33 on the reaction container table 190 and dispenses the aspirated sample into the reaction container 33 at the second sample discharging position 161 on the transfer unit 160.

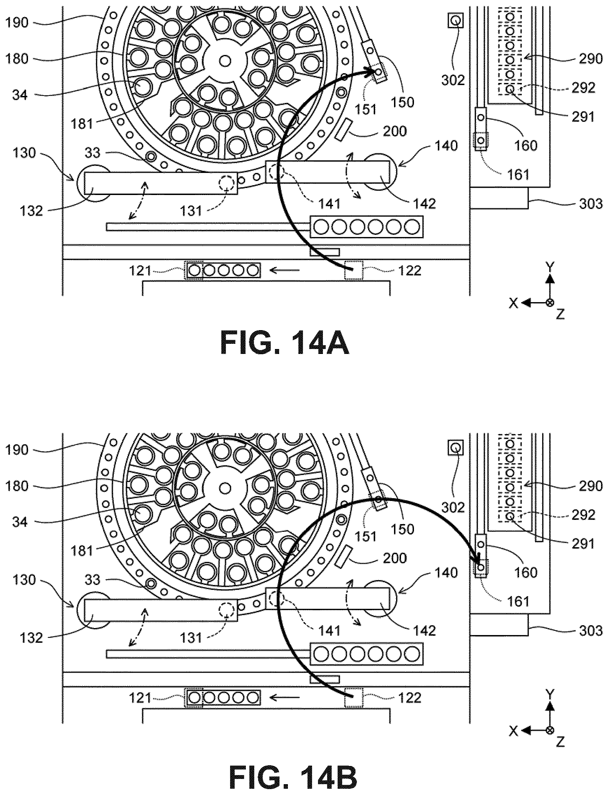

When the aspirating mode set for the sample is the second aspirating mode, as illustrated in FIG. 14A, the sample dispensing unit 140 aspirates the sample from the sample container 32 at the sample aspirating position 122 and dispenses the aspirated sample into the reaction container 33 at the first sample discharging position 151 on the transfer unit 150. Alternatively, as illustrated in FIG. 14B, the sample dispensing unit 140 aspirates the sample from the sample container 32 at the sample aspirating position 122 and dispenses the aspirated sample into the reaction container 33 at the second sample discharging position 161 on the transfer unit 160.

Whether the sample dispensing unit 140 dispenses the aspirated sample into the reaction container 33 on the transfer unit 150 or the reaction container 33 on the transfer unit 160 is determined depending on the measurement item set for the sample as described later. When the sample dispensing unit 140 discharges the sample into the reaction container 33 on the transfer unit 150, the reagent dispensing unit 240 and the detection unit 280 perform the reagent dispensing and the measurement for this reaction container 33 as described later. Meanwhile, when the sample dispensing unit 140 discharges the sample into the reaction container 33 on the transfer unit 160, the reagent dispensing unit 250 and the detection unit 290 perform the reagent dispensing and the measurement for this reaction container 33 as described later.

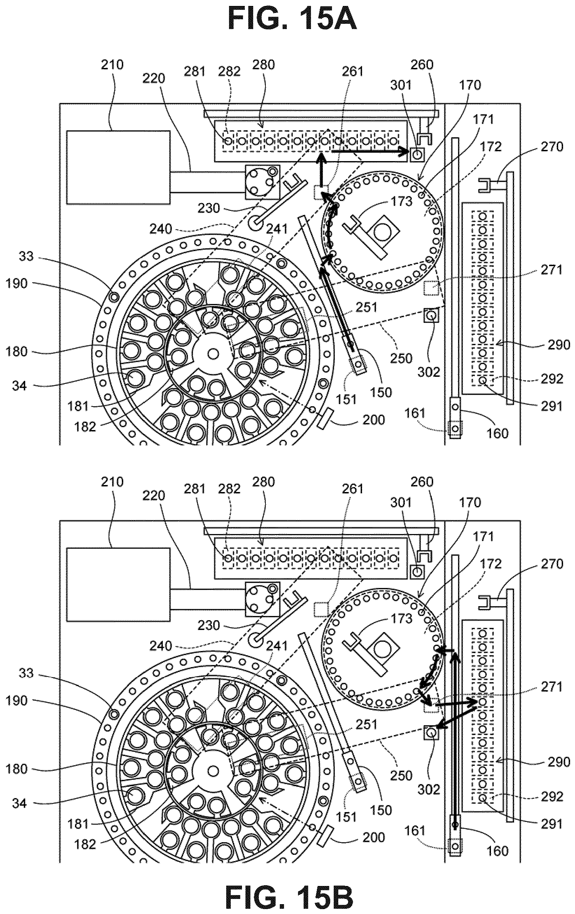

When the sample dispensing unit 140 has discharged the sample into the reaction container 33 on the transfer unit 150, as illustrated in FIG. 15A, the transfer unit 150 transfers the reaction container 33 to a position near the heating table 170. The transfer unit 173 transfers the reaction container 33 transferred to the position near the heating table 170 to place it in the heating table 170. When the adjustment reagent is to be dispensed into the reaction container 33, the transfer unit 173 transfers the reaction container 33 to the first discharging position 261 and the reagent dispensing unit 240 dispenses the adjustment reagent into the reaction container 33.

After the completion of heating in the heating table 170, the heating table 170 is rotated to transfer the reaction container 33 to a position near the first discharging position 261. The transfer unit 260 transfers the reaction container 33 to the first discharging position 261. The reagent dispensing unit 240 dispenses the trigger reagent into the reaction container 33 at the first discharging position 261. Thereafter, the transfer unit 260 transfers the reaction container 33 to one of the holders 281 of the detection unit 280 and sets the reaction container 33 therein. The detector 282 performs the measurement on the reaction container 33 set in the holder 281 and outputs the detection signal to the information processing apparatus 103. When the measurement is completed, the transfer unit 260 transfers the reaction container 33 to the disposal opening 301 to dispose of the reaction container 33 into the discarding unit 303.

When the sample dispensing unit 140 has discharged the sample into the reaction container 33 on the transfer unit 160, as illustrated in FIG. 15B, the transfer unit 160 transfers the reaction container 33 to a position near the heating table 170. The transfer unit 270 transfers the reaction container 33 transferred to the position near the heating table 170 to place it in the heating table 170. When the adjustment reagent is to be dispensed into the reaction container 33, the transfer unit 173 transfers the reaction container 33 to the second discharging position 271 and the reagent dispensing unit 250 dispenses the adjustment reagent into the reaction container 33.

After the completion of heating in the heating table 170, the heating table 170 is rotated to transfer the reaction container 33 to a position near the second discharging position 271. The transfer unit 270 transfers the reaction container 33 to the second discharging position 271. The reagent dispensing unit 250 dispenses the trigger reagent into the reaction container 33 at the second discharging position 271. Thereafter, the transfer unit 270 transfers the reaction container 33 to one of the holders 291 of the detection unit 290 and sets the reaction container 33 therein. The detector 292 performs the measurement on the reaction container 33 set in the holder 291 and outputs the detection signal to the information processing apparatus 103. When the measurement is completed, the transfer unit 270 transfers the reaction container 33 to the disposal opening 302 to dispose of the reaction container 33 into the discarding unit 303.

Note that the reagent dispensing unit 250 and the detection unit 290 may perform the reagent dispensing and the measurement on the reaction container 33 transferred by the transfer unit 150 and then set in the heating table 170. Moreover, the reagent dispensing unit 240 and the detection unit 280 may perform the reagent dispensing and the measurement on the reaction container 33 transferred by the transfer unit 160 and then set in the heating table 170.

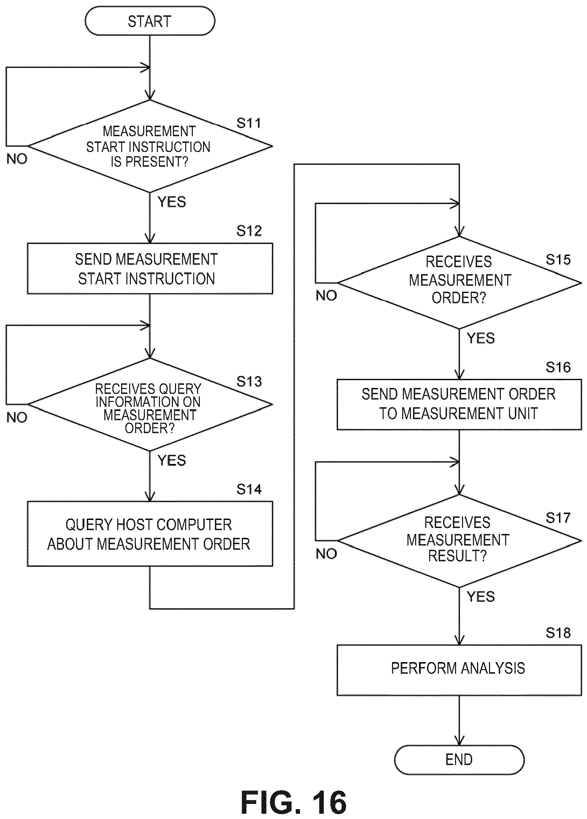

Processing of the information processing apparatus 103 is described with reference to the flowchart of FIG. 16.

As illustrated in FIG. 16, when the controller 103a of the information processing apparatus 103 receives a measurement start instruction from the user via the input unit 103d in step S11, the controller 103a sends the measurement start instruction to the measurement unit 101 in step S12. In response to this, the controller 101a of the measurement unit 101 reads the bar codes from the sample rack 31 and the sample containers 32 and obtains the identification information of the sample rack 31 and the identification information of the sample containers 32 from the read bar codes as described later. The controller 101a sends query information on a measurement order including the identification information to the information processing apparatus 103.

When the controller 103a receives the query information on the measurement order from the measurement unit 101 in step S13, the controller 103a sends the received query information on the measurement order to a host computer in step S14. When the host computer receives the query information on the measurement order, the host computer reads the measurement item set in advance for each sample from a memory of the host computer, based on the identification information included in the received information, and sends the measurement order including the identification information of each sample container 32 and the setting item set for the sample in the sample container 32 to the information processing apparatus 103.

When the controller 103a receives the measurement order from the host computer in step S15, the controller 103a sends the received measurement order to the measurement unit 101 in step S16. Measurement corresponding to the measurement item set for each sample is performed on the sample in the measurement unit 101 as described later. When the controller 103a receives the measurement result from the measurement unit 101 in step S17, the controller 103a stores the received measurement result in the memory 103b and, in step S18, analyzes the sample based on the received measurement result and the measurement item set for the sample. The measurement result received by the controller 103a in step S17 includes the signals detected based on the light having all wavelengths. Accordingly, in step S18, the controller 103a analyzes the sample based on only the signal necessary for the measurement item. The controller 103a stores the analysis result in the memory 103b.

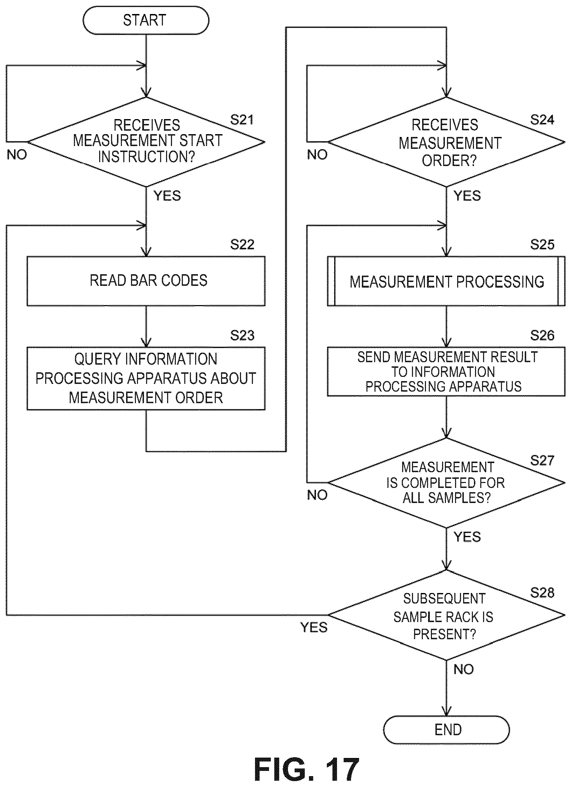

Processing of the measurement unit 101 is described with reference to the flowcharts of FIGS. 17 to 20.

As illustrated in FIG. 17, when the controller 101a of the measurement unit 101 receives the measurement start instruction from the information processing apparatus 103 in step S21, the controller 101a reads the bar codes from the sample rack 31 and the sample containers 32 in step S22. The controller 101a thereby obtains the identification information of the sample rack 31 and the identification information of the sample containers 32. In step S23, the controller 101a sends the query information on the measurement order including the identification information to the information processing apparatus 103 and query about the measurement order.

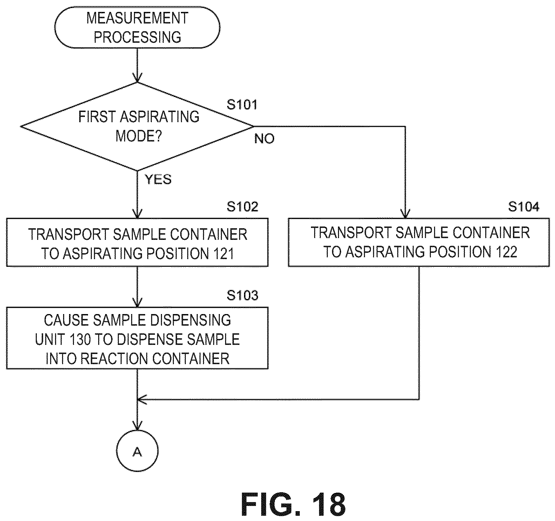

When the controller 101a receives the measurement order from the information processing apparatus 103 in step S24, the controller 101a performs measurement processing for the target sample based on the received measurement order in step S25. The measurement processing is described later with reference to FIGS. 18 to 20. In step S26, the controller 101a sends the measurement result obtained in the measurement processing to the information processing apparatus 103. In step S27, the controller 101a performs processing of steps S25, S26 until the measurement is completed for all samples held in the sample rack 31. In step S28, the controller 101a determines whether there is a subsequent sample rack 31 in the transporter 102. When there is a subsequent sample rack 31, the processing returns to step S22. When there is no subsequent sample rack 31, the processing illustrated in FIG. 17 is completed.

As illustrated in FIG. 18, in step S101, the controller 101a determines in which one of the first aspirating mode and the second aspirating mode the target sample is to be aspirated, based on the received measurement order for the target sample. When the aspirating mode is determined to be the first aspirating mode, in step S102, the controller 101a causes the sample container 32 to be transported to the sample aspirating position 121. In step S103, the controller 101a causes the sample dispensing unit 130 to aspirate the sample from the sample container 32 at the sample aspirating position 121 by an amount necessary for performing measurement twice and discharge the sample into the new reaction container 33 on the reaction container table 190. When the aspirating mode is determined to be the second aspirating mode, in step S104, the controller 101a causes the sample container 32 to be transported to the sample aspirating position 122.

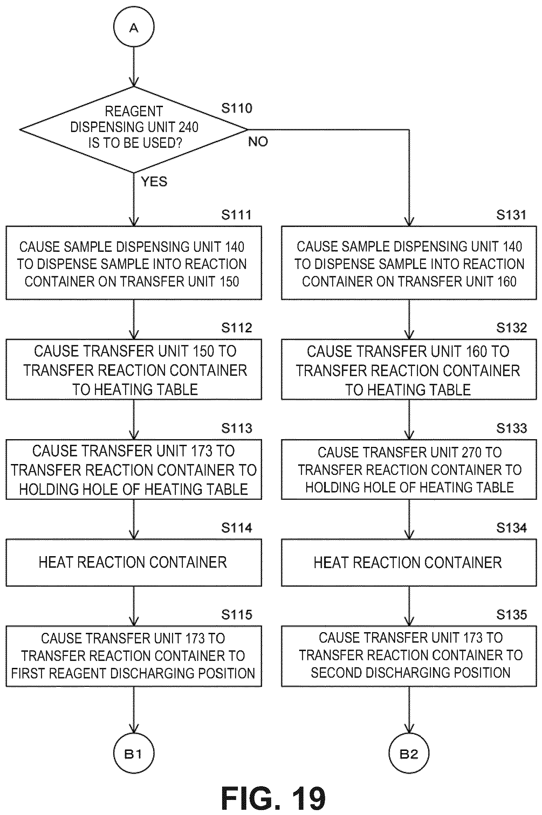

As illustrated in FIG. 19, in step S110, the controller 101a determines which one of the reagent dispensing unit 240 and the reagent dispensing unit 250 is to be used in the dispensing of the reagent into the target sample, based on the received measurement order for the target sample. The reagent dispensing unit to be used in the dispensing of the reagent is set in advance for each measurement item and the association information in which the measurement items and the reagent dispensing units to be used in the dispensing of the reagent are associated with one another is stored in the memory 101b of the measurement unit 101 as described with reference to FIG. 12B.

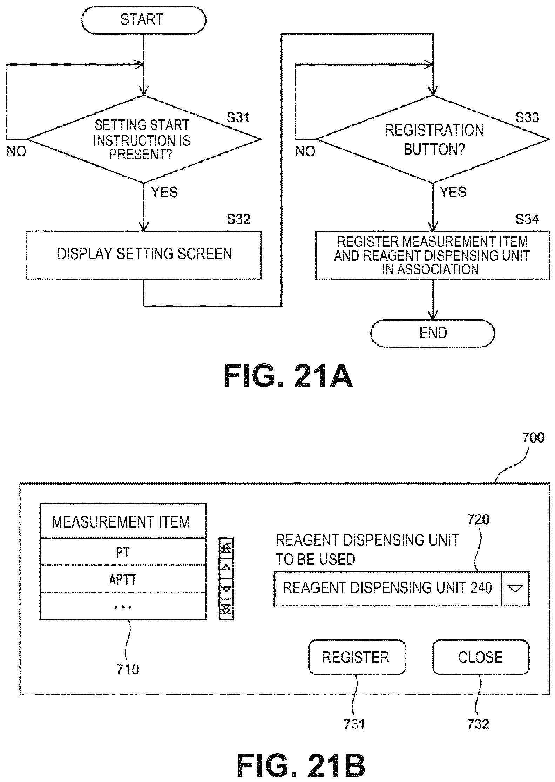

For example, as illustrated in FIG. 12B, assume that the association information includes information indicating that the reagent dispensing unit 240 is used when the measurement item is PT and information indicating that the reagent dispensing unit 250 is used when the measurement item is APTT. In this case, when the measurement item of the target sample is PT, the controller 101a sets the reagent dispensing unit 240 as the reagent dispensing unit to be used in the reagent dispensing for the target sample. Meanwhile, when the measurement item of the target sample is APTT, the controller 101a sets the reagent dispensing unit 250 as the reagent dispensing unit to be used in the reagent dispensing for the target sample. Setting of the association information is described later with reference to FIGS. 21A and 21B.

Description is given of the case where the reagent dispensing unit to be used in the reagent dispensing for the target sample is the reagent dispensing unit 240 and the case where the reagent dispensing unit to be used is the reagent dispensing unit 250, one by one. Moreover, for the sake of convenience, description is given of the case where the reagent dispensing unit discharges two types of reagents into the reaction container 33, that is the case where the adjustment reagent and the trigger reagent are used.

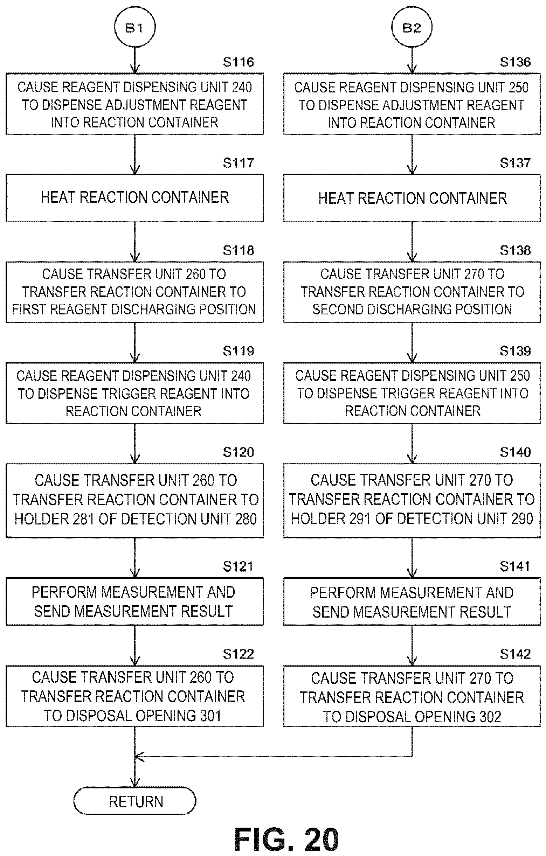

When the reagent dispensing unit to be used in the reagent dispensing for the target sample is the reagent dispensing unit 240, processing of the steps S111 to S122 in FIGS. 19 and 20 are performed. In step S111, the controller 101a causes the sample dispensing unit 140 to dispense the target sample into the new reaction container 33 on the transfer unit 150. Specifically, when the aspirating mode for the target sample is the first aspirating mode, the sample dispensing unit 140 aspirates the target sample from the reaction container 33 on the reaction container table 190 and discharges the target sample into the reaction container 33 on the transfer unit 150. In this case, the target sample of an amount necessary for re-examination is left in the reaction container 33 on the reaction container table 190. When the re-examination is performed, the target sample in this reaction container 33 is used. When the aspirating mode for the target sample is the second aspirating mode, the sample dispensing unit 140 aspirates the sample from the sample container 32 at the sample aspirating position 122 by an amount necessary for performing measurement once and discharges the sample into the reaction container 33 on the transfer unit 150.

In step S112, the controller 101a causes the transfer unit 150 to transfer the reaction container 33 to a position near and on the left side of the heating table 170. In step S113, the controller 101a causes the transfer unit 173 of the heating table 170 to transfer the reaction container 33 on the transfer unit 150 to one of the holding holes 171 of the heating table 170. In step S114, the controller 101a causes the heater 172 to heat the target reaction container 33. In step S115, the controller 101a causes the transfer unit 173 to transfer the target reaction container 33 to the first discharging position 261.

As illustrated in FIG. 20, in step S116, the controller 101a causes the reagent dispensing unit 240 to dispense the adjustment reagent into the target reaction container 33 disposed at the first discharging position 261. In step S117, the controller 101a causes the transfer unit 173 to transfer the target reaction container 33 to the holding hole 171 of the heating table 170 and cause the heater 172 to heat the target reaction container 33. In step S118, the controller 101a causes the transfer unit 260 to transfer the target reaction container 33 to the first discharging position 261. In step S119, the controller 101a causes the reagent dispensing unit 240 to dispense the trigger reagent into the target reaction container 33 disposed at the first discharging position 261.

In step S120, the controller 101a causes the transfer unit 260 to transfer the reaction container 33 into which the trigger reagent is dispensed to the holder 281 of the detection unit 280. In step S121, the controller 101a measures the measurement specimen in the target reaction container 33 held by the holder 281 and sends the measurement result to the information processing apparatus 103. Specifically, the controller 101a causes the light emitting unit 400 to emit the light having various wavelengths to the reaction container 33 and cause the sensor 602 of the detector 282 to receive the light generated in each wavelength. Then, the controller 101a obtains the output signals of the sensor 602 and sends the output signals to the information processing apparatus 103.

In step S122, the controller 101a causes the transfer unit 260 to transfer the target reaction container 33 for which the measurement is completed to the disposal opening 301 and dispose of the target reaction container 33. The measurement processing for the target sample is thereby completed.

Returning to FIG. 19, when the reagent dispensing unit to be used in the reagent dispensing for the target sample is the reagent dispensing unit 250, processing of the steps S131 to S142 in FIGS. 19 and 20 are performed. In step S131, the controller 101a causes the sample dispensing unit 140 to dispense the target sample into the new reaction container 33 on the transfer unit 160. Also in this case, when the aspirating mode for the target sample is the first aspirating mode, the sample dispensing unit 140 aspirates the target sample from the reaction container 33 on the reaction container table 190 and discharges the target sample into the reaction container 33 on the transfer unit 160. When the aspirating mode for the target sample is the second aspirating mode, the sample dispensing unit 140 aspirates the sample from the sample container 32 at the sample aspirating position 122 by an amount necessary for performing measurement once and discharges the sample into the reaction container 33 on the transfer unit 160.

In step S132, the controller 101a causes the transfer unit 160 to transfer the reaction container 33 to a position near and on the right side of the heating table 170. In step S133, the controller 101a causes the transfer unit 270 to transfer the reaction container 33 on the transfer unit 160 to one of the holding holes 171 of the heating table 170. In step S134, the controller 101a causes the heater 172 to heat the target reaction container 33. In step S135, the controller 101a causes the transfer unit 173 to transfer the target reaction container 33 to the second discharging position 271.

As illustrated in FIG. 20, in step 136, the controller 101a causes the reagent dispensing unit 250 to dispense the adjustment reagent into the target reaction container 33 disposed at the second discharging position 271. In step S137, the controller 101a causes the transfer unit 173 to transfer the target reaction container 33 to the holding hole 171 of the heating table 170 and cause the heater 172 to heat the target reaction container 33. In step S138, the controller 101a causes the transfer unit 270 to transfer the target reaction container 33 to the second discharging position 271. In step S139, the controller 101a causes the reagent dispensing unit 250 to dispense the trigger reagent into the target reaction container 33 disposed at the second discharging position 271.

In step S140, the controller 101a causes the transfer unit 270 to transfer the reaction container 33 into which the trigger reagent is dispensed to the holder 291 of the detection unit 290. In step S141, the controller 101a measures the measurement specimen in the target reaction container 33 held by the holder 291 and sends the measurement result to the information processing apparatus 103. Specifically, the controller 101a causes the light emitting unit 400 to emit the light having various wavelengths to the reaction container 33 and cause the sensor 612 of the detector 292 to receive the light generated in each wavelength. Then, the controller 101a obtains the output signals of the sensor 612 and sends the output signals to the information processing apparatus 103.

In step S142, the controller 101a causes the transfer unit 270 to transfer the target reaction container 33 for which the measurement is completed to the disposal opening 302 and dispose of the target reaction container 33. The measurement processing for the target sample is thereby completed.

Note that, when no adjustment reagent is used and only the trigger reagent is used, steps S115 to step S117 and steps S135 to S137 are omitted.

As described above, the reagent dispensing unit to be used in the dispensing of the reagent is determined to be one of the reagent dispensing units 240, 250 and the detection unit to be used for the measurement is determined to be one of the detection units 280, 290, depending on the measurement item. The measurement results with substantially the same level of accuracy are thereby obtained for the same measurement item. Moreover, for the same measurement item, the same reagent dispensing unit is used for the dispensing of the reagent and the same detection unit is used for the measurement. Thus, there is no need to perform both of preparation work such as quality control performed when the reagent dispensing unit 240 and the detection unit 280 are to be used and preparation work such as quality control performed when the reagent dispensing unit 250 and the detection unit 290 are to be used. The preparation work by the user can be thereby simplified.

Next, setting of the association information in which the measurement items and the reagent dispensing units to be used for the dispensing of the reagents are associated with one another is described with reference to FIGS. 21A and 21B.

In a facility in which the sample analyzer 100 is installed, the user sets the association information depending on conditions of the measurement items to be processed in the facility. For example, if one of the reagent dispensing units is intensively used in measurement corresponding to a measurement item occurring with high frequency, the processing efficiency of the sample analyzer 100 may decrease. In such a case, the user sets the association information such that the measurement corresponding to the measurement item occurring with high frequency is distributed to the two reagent dispensing units.

As illustrated in FIG. 21A, when the controller 103a receives a setting start instruction from the user via the input unit 103d in step S31, the controller 103a displays a receiving portion 700 for receiving setting of the reagent dispensing unit to be used for each measurement item, on the display 103c in step S32.

As illustrated in FIG. 21B, the receiving portion 700 is a screen which receives inputs from the user via the input unit 103d and which displays information. The receiving portion 700 includes a measurement item display region 710, a reagent dispensing unit display region 720, and a registration button 731, and a close button 732. The measurement item display region 710 is configured to display a list of the measurement items measureable by the sample analyzer 100 and allow the user to select any of the measurement items via the input unit 103d. The reagent dispensing unit display region 720 is configured to display the name of one of the reagent dispensing units 240, 250 and allow the user to select one of the reagent dispensing units 240, 250 via the input unit 103d.

When the user presses the registration button 731 by using the input unit 103d, the receiving portion 700 is closed and the association information is registered. When the user presses the close button 732 by using the input unit 103d, the receiving portion 700 is closed without the association information being registered.

Returning to FIG. 21A, when the controller 103a determines that the registration button 731 is pressed in step S33, the controller 103a registers the measurement item and the reagent dispensing unit in association in step S34, according to the setting received via the receiving portion 700. Specifically, the controller 103a stores information in which the measurement item selected in the measurement item display region 710 and the reagent dispensing unit set in the reagent dispensing unit display region 720 are associated with each other, in the memory 103b. Then, the controller 103a sends the association information stored in the memory 103b to the measurement unit 101. The measurement unit 101 stores the received association information in the memory 101b. In step S110 of FIG. 19, the controller 101a of the measurement unit 101 can thereby determine which one of the reagent dispensing unit 240 and the reagent dispensing unit 250 to use, based on the measurement order for the target sample and the association information stored in the memory 101b.

For example, when the user presses the registration button 731 with the measurement item PT selected in the measurement item display region 710 and with the reagent dispensing unit 240 selected in the reagent dispensing unit display region 720, the association information in which the measurement item PT and the reagent dispensing unit 240 are associated with each other is stored in the memorys 103b, 101b. The reagent dispensing unit 240 is thereby used to dispense the reagent when the measurement item for the target sample is PT.

As described above, since the sample analyzer 100 can receive the setting of the reagent dispensing unit to be used for each measurement item via the receiving portion 700, the user can arbitrary select the reagent dispensing unit to be used for each measurement item, depending on the conditions of the measurement items to be processed in the facility. The processing efficiency of the sample analyzer 100 can be thereby improved depending on the conditions of the measurement items to be processed in the facility.

### DESCRIPTION OF REFERENCE NUMERALS

10 sample analyzer
11 heating table
11a holding hole
11b heater
12 first reagent dispensing unit
13 second reagent dispensing unit
14 first detection unit
14a first holder
14b first detector
15 second detection unit
15a second holder
15b second detector
16 first transfer unit
17 second transfer unit
18 first discarding transfer unit
19 second discarding transfer unit
20 first sample transfer unit
21 second sample transfer unit
22 sample dispensing unit
23 reagent table
23a first aspirating position
23b second aspirating position
24 discarding unit
25 transporter
26 controller
26a first controller
26b second controller
32 sample container
33 reaction container
34 reagent container
100 sample analyzer
101a, 103a controller
102 transporter
103c cooling device
121, 122 sample aspirating position
130, 140 sample dispensing unit
150 transfer unit
151 first sample discharging position
160 transfer unit
161 second sample discharging position
170 heating table
171 holding hole
172 heater
174 first position
175 second position
180 reagent table
201 support unit
240, 250 reagent dispensing unit
241 first aspirating position
251 second aspirating position
244 first pipet
245 first heater
254 second pipet
255 second heater
260 transfer unit
261 first discharging position
270 transfer unit
271 second discharging position
280, 290 detection unit
281, 291 holder
282, 292 detector
303 discarding unit
400 light emitting unit
410 light source
471 first optical fiber
472 second optical fiber
602, 612 sensor
700 receiving portion

## Claims

1. A sample analyzer comprising:
a reagent table on which reagent containers each holding a reagent are placed and that is configured to transfer the placed reagent containers to a first aspirating position and a second aspirating position;
a first reagent dispensing unit that aspirates the reagent from the reagent container transferred to the first aspirating position, heats the aspirated reagent, and dispenses the heated reagent into a reaction container;
a second reagent dispensing unit that aspirates the reagent from the reagent container transferred to the second aspirating position, heats the aspirated reagent, and dispenses the heated reagent into a reaction container;
a detection unit comprising:
holders that hold reaction containers each holding a measurement specimen containing a sample and the reagent dispensed by the first reagent dispensing unit and reaction containers each holding a measurement specimen containing a sample and the reagent dispensed by the second reagent dispensing unit, and
detectors that are provided for the holders, respectively; and
a controller that causes the reagent table to:
transfer the reagent container, which holds the reagent relating to a measurement item for which the first reagent dispensing unit is set to be used, to the first aspirating position; and
transfer the reagent container, which holds the reagent relating to a measurement item for which the second reagent dispensing unit is set to be used, to the second aspirating position.

2. The sample analyzer according to claim 1, wherein
the detection unit comprises:
a first detection unit; and
a second detection unit,
the first detection unit comprises:
first holders configured to hold the reaction containers each holding the measurement specimen containing the sample and the reagent dispensed by the first reagent dispensing unit; and
first detectors provided for the first holders, respectively, and
the second detection unit comprises:
second holders configured to hold the reaction containers each holding the measurement specimen containing the sample and the reagent dispensed by the second reagent dispensing unit; and
second detectors provided for the second holders, respectively.

3. The sample analyzer according to claim 2, further comprising a receiving portion that receives setting in which one of the first reagent dispensing unit and the second reagent dispensing unit is set to be used for each measurement item, wherein
the controller controls the reagent table depending on the setting received via the receiving portion.

4. The sample analyzer according to claim 2 or 3, wherein
the first reagent dispensing unit comprises a first pipet and a first heater that heats the reagent held by the first pipet, and
the second reagent dispensing unit comprises a second pipet and a second heater that heats the reagent held by the second pipet.

5. The sample analyzer according to any one of claims 2 to 4, further comprising a cooling device that cools the reagent containers placed on the reagent table.

6. The sample analyzer according to any one of claims 2 to 5, wherein
the first detectors detect signals for analysis from the measurement specimens in the reaction containers into which the reagent is dispensed by the first reagent dispensing unit and that are held by the respective first holders,
the second detectors detect signals for analysis from the measurement specimens in the reaction containers into which the reagent is dispensed by the second reagent dispensing unit and that are held by the respective second holders, and
the controller analyzes the samples in the reaction containers held by the first holders based on the signals detected by the first detectors and analyzes the samples in the reaction containers held by the second holders based on the signals detected by the second detectors.

7. The sample analyzer according to claim 6, further comprising a light emitting unit comprising:
a light source unit;
first optical fibers that guide light emitted from the light source unit to the first detectors; and
second optical fibers that guide the light emitted from the light source unit to the second detectors.

8. The sample analyzer according to claim 7, wherein the light source unit emits at least one of:
light having a first wavelength for blood coagulation time measurement;
light having a second wavelength for synthetic substrate measurement; and
light having a third wavelength for immunonephelometry measurement.

9. The sample analyzer according to claim 7 or 8, wherein the light source unit emits:
light having a first wavelength for blood coagulation time measurement;
light having a second wavelength for synthetic substrate measurement; and
light having a third wavelength for immunonephelometry measurement.

10. The sample analyzer according to any one of claims 7 to 9, wherein
the light source unit sequentially and repeatedly emits:
light having a first wavelength;
light having a second wavelength; and
light having a third wavelength,
the controller generates time-series data for each of the light having the first wavelength, the light having the second wavelength, and the light having the third wavelength and analyzes the samples based on the generated time-series data, and
the light having the first wavelength, the light having the second wavelength, and the light having the third wavelength are each one of light for blood coagulation time measurement, light for synthetic substrate measurement, and light for immunonephelometry measurement,.

11. The sample analyzer according to any one of claims 7 to 10, wherein
the first detectors comprise sensors that receive light from the reaction containers held by the first holders and output the signals for analysis, respectively,
the second detectors comprise sensors that receive light from the reaction containers held by the second holders and output the signals for analysis, respectively, and
the controller analyzes the samples in the reaction containers held by the first holders based on changes with time in the signals outputted by the sensors of the first detectors and analyzes the samples in the reaction containers held by the second holders based on changes with time in the signals outputted by the sensors of the second detectors.

12. The sample analyzer according to any one of claims 6 to 11, wherein the controller calculates a coagulation time based each of the signals detected by the first detectors and calculates a coagulation time based on each of the signals detected by the second detectors.

13. The sample analyzer according to any one of claims 2 to 12, further comprising:
a heating table comprising:
holding holes in which the reaction containers holding the samples are held, respectively; and
a heater that heats the reaction containers held in the holding holes,
a first transfer unit that transfers one of the reaction containers held in the holding holes of the heating table to one of the first holders, and
a second transfer unit that transfers one of the reaction containers held in the holding holes of the heating table to one of the second holders.

14. The sample analyzer according to claim 13, wherein
the first transfer unit transfers the reaction container held in the holding hole at a first position to the first holder, and
the second transfer unit transfers the reaction container held in the holding hole at a second position different from the first position to the second holder.

15. The sample analyzer according to claim 13 or 14, wherein
the first reagent dispensing unit dispenses the reagent into the reaction container at a first discharging position on a path in which the reaction container is transferred by the first transfer unit, and
the second reagent dispensing unit dispenses the reagent into the reaction container at a second discharging position on a path in which the reaction container is transferred by the second transfer unit.

16. The sample analyzer according to claim 15, wherein a transfer distance by which the first transfer unit transfers the reaction container from the first discharging position to the first detection unit is substantially equal to a transfer distance by which the second transfer unit transfers the reaction container from the second discharging position to the second detection unit.

17. The sample analyzer according to any one of claims 13 to 16, further comprising a housing comprising a first side surface and a second side surface adjacent to the first side surface, wherein
the heating table is arranged inside the housing and near an adjacent part in which the first side surface is adjacent to the second side surface,
the first detection unit is arranged on a side of the first side surface close to the adjacent part and along the first side surface of the housing,
the second detection unit is arranged on a side of the second side surface close to the adjacent part and along the second side surface of the housing,
the first holders of the first detection unit are arranged side by side along the first side surface in a plan view, and
the second holders of the second detection unit are arranged side by side along the second side surface in the plan view.

18. The sample analyzer according to claim 17, wherein a direction in which the first holders are arranged side by side is substantially orthogonal to a direction in which the second holders are arranged side by side.

19. The sample analyzer according to claim 17 or 18, further comprising:
a transporter that transports a sample container holding the sample, to a sample aspirating position; and
a sample dispensing unit that dispenses, into the reaction containers, the sample in the sample container transferred by the transporter, wherein
the housing further comprises a third side surface not adjacent to the first side surface but adjacent to the second side surface, and
the transporter is arranged along the third side surface of the housing.

20. The sample analyzer according to one of claims 13 to 19, further comprising:
a discarding unit configured to house one or more reaction containers to be discarded;
a first discarding transfer unit that transfers, to the discarding unit, the reaction container held by the first holder and to be discarded; and
a second discarding transfer unit that transfers, to the discarding unit, the reaction container held by the second holder and to be discarded.

21. The sample analyzer according to any one claims 13 to 20, further comprising:
a transporter that transports a sample container holding the sample, to a sample aspirating position;
a sample dispensing unit that:
aspirates the sample from the sample container transported to the sample aspirating position and dispenses the aspirated sample into the reaction container at a first sample discharging position, and
aspirates the sample from the sample container transported to the sample aspirating position and dispenses the aspirated sample into the reaction container at a second sample discharging position;
a first sample transfer unit that transfers, from the first sample discharging position to the heating table, the reaction container into which the sample is dispensed by the sample dispensing unit; and
a second sample transfer unit that transfers, from the second sample discharging position to the heating table, the reaction container into which the sample is dispensed by the sample dispensing unit.

22. The sample analyzer according to claim 21, further comprising a transporter that transports the sample container holding the sample to a aspirating position, wherein
the sample dispensing unit aspirates the sample from the sample container transported to the aspirating position by the transporter and dispenses the aspirated sample into the reaction container.

23. The sample analyzer according to any one of claims 1 to 22, wherein the first reagent dispensing unit and the second reagent dispensing unit are supported by a support unit arranged above the reagent table.

24. The sample analyzer according to any one of claims 1 to 23, wherein the controller comprises:
a first controller that controls a mechanism for measurement of the measurement specimens; and
a second controller that analyzes the samples.

25. The sample analyzer according to any one of claims 1 to 24, wherein the samples comprise plasma or serum.
